(19) Europäisches Patentamt
European Patent Office
Office européen des brevets

(11) EP 2 436 382 A1

(12) # EUROPEAN PATENT APPLICATION

(43) Date of publication:
**04.04.2012 Bulletin 2012/14**

(21) Application number: **11195407.9**

(22) Date of filing: **24.09.2007**

(51) Int Cl.:
***A61K 31/16*** *(2006.01)* ***A61K 31/295*** *(2006.01)*
***A61K 33/26*** *(2006.01)* ***A61K 33/30*** *(2006.01)*
***A61P 35/00*** *(2006.01)*

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **28.09.2006 US 84830006 P**

(62) Document number(s) of the earlier application(s) in accordance with Art. 76 EPC:
**07861361.9 / 2 079 462**

(71) Applicant: **Merck Sharp & Dohme Corporation**
**Rahway, NJ 07065 (US)**

(72) Inventors:
- **MCKEOWN, Arlene, E.**
  **Rahway, NJ New Jersey 07065-0907 (US)**
- **MILLER, Thomas, A.**
  **Rahway, NJ New Jersey 07065-0907 (US)**

(74) Representative: **Hussain, Deeba**
**Merck & Co., Inc.**
**Patent Department**
**Hertford Road**
**Hoddesdon**
**Hertfordshire EN11 9BU (GB)**

Remarks:
This application was filed on 22-12-2011 as a divisional application to the application mentioned under INID code 62.

(54) **Pharmaceutical compositions of HDAC inhibitors and chelatable metal compounds, and metal-HDAC inhibitor chelate complexes**

(57) The present invention provides pharmaceutical compositions of SAHA and a chelatable metal compound comprising zinc or iron. In one embodiment, the invention provides a method of treating cancer and alleviating the side effects of SAHA by administering the pharmaceutical composition. In another embodiment, the present invention also provides pharmaceutical compositions of zinc SAHA chelate complexes or iron SAHA chelate complexes. In another embodiment, the invention provides methods of treating cancer by administering the pharmaceutical compositions.

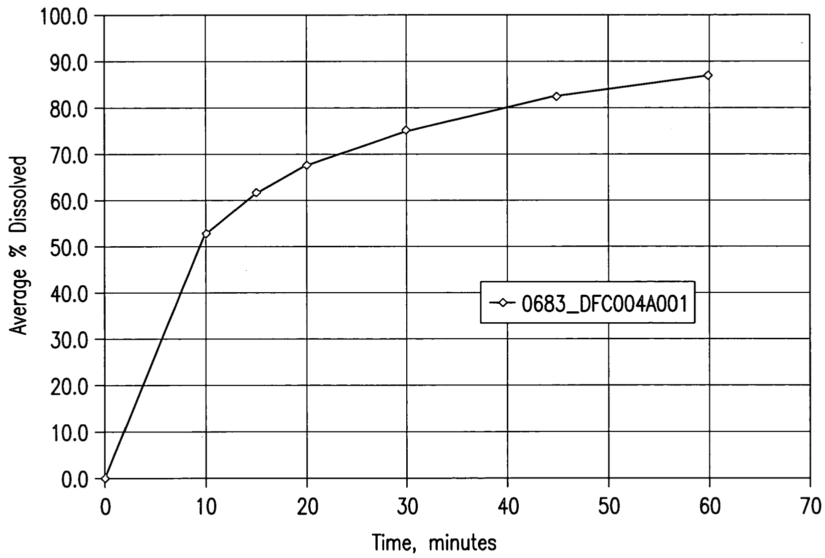


FIG.1



Printed by Jouve, 75001 PARIS (FR)

**Description**

FIELD OF THE INVENTION

The present invention provides pharmaceutical compositions of an HDAC inhibitor and a chelatable metal compound. In one embodiment, the invention provides a method of treating cancer and alleviating the side effects of the HDAC inhibitor by administering the pharmaceutical composition. In another embodiment, the present invention also provides pharmaceutical compositions of metal HDAC inhibitor chelate complexes. In another embodiment, the invention provides methods of treating cancer by administering the pharmaceutical compositions. The invention provides crystalline compositions of metal HDAC inhibitor chelate complexes and methods of producing same.

BACKGROUND OF THE INVENTION

Throughout this application various publications are referenced by arabic numerals within parentheses. Full citations for these publications may be found at the end of the specification immediately preceding the claims.

Cancer is a disorder in which a population of cells has become, in varying degrees, unresponsive to the control mechanisms that normally govern proliferation and differentiation. For many years there have been two principal strategies for chemotherapeutic treatment of cancer: a) blocking hormone-dependent tumor cell proliferation by interference with the production or peripheral action of sex hormones; and b) killing cancer cells directly by exposing them to cytotoxic substances, which injure both neoplastic and normal cell populations.

Cancer therapy is also being attempted by the induction of terminal differentiation of the neoplastic cells (1). In cell culture models differentiation has been reported by exposure of cells to a variety of stimuli, including: cyclic AMP and retinoic acid (2,3), aclarubicin and other anthracyclines (4).

Despite many advances in the field of oncology, the majority of solid tumors remain incurable in the advanced stages. Cytotoxic therapy is used in most cases, however, it often causes significant morbidity to the patient without significant clinical benefit. Less toxic and more specific agents to treat and control advanced malignancies are being explored.

There is abundant evidence that neoplastic transformation does not necessarily destroy the potential of cancer cells to differentiate (1,5,6). There are many examples of tumor cells which do not respond to the normal regulators of proliferation and appear to be blocked in the expression of their differentiation program, and yet can be induced to differentiate and cease replicating. A variety of agents, including some relatively simple polar compounds (5,7-9), derivatives of vitamin D and retinoic acid (10-12), steroid hormones (13), growth factors (6,14), proteases (15,16), tumor promoters (17,18), and inhibitors of DNA or RNA synthesis (4,19-24), can induce various transformed cell lines and primary human tumor explants to express more differentiated characteristics.

Histone deacetylase inhibitors such as suberoylanilide hydroxamide acid (SAHA), belong to this class of agents that have the ability to induce tumor cell growth arrest, differentiation and/or apoptosis (25). These compounds are targeted towards mechanisms inherent to the ability of a neoplastic cell to become malignant, as they do not appear to have toxicity in doses effective for inhibition of tumor growth in animals (26). There are several lines of evidence that histone acetylation and deacetylation are mechanisms by which transcriptional regulation in a cell is achieved (27). These effects are thought to occur through changes in the structure of chromatin by altering the affinity of histone proteins for coiled DNA in the nucleosome. There are five types of histones that have been identified in nucleosomes (designated H1, H2A, H2B, H3 and H4). Each nucleosome contains two of each histone type within its core, except for H1, which is present singly in the outer portion of the nucleosome structure. It is believed that when the histone proteins are hypoacetylated, there is a greater affinity of the histone to the DNA phosphate backbone. This affinity causes DNA to be tightly bound to the histone and renders the DNA inaccessible to transcriptional regulatory elements and machinery. The regulation of acetylated states occurs through the balance of activity between two enzyme complexes, histone acetyl transferase (HAT) and histone deacetylase (HDAC). The hypoacetylated state is thought to inhibit transcription of associated DNA. This hypoacetylated state is catalyzed by large multiprotein complexes that include HDAC enzymes. In particular, HDACs have been shown to catalyze the removal of acetyl groups from the chromatin core histones.

SAHA (ZOLINZA ™ (vorinostat)) has been shown to be useful for treating cancer, selectively inducing terminal differentiation of neoplastic cells, inducing cell growth arrest and/or inducing apoptosis. The inhibition of HDAC by SAHA is thought occur through direct interaction with the catalytic site of the enzyme as demonstrated by X-ray crystallography studies (28). The result of HDAC inhibition is not believed to have a generalized effect on the genome, but rather, only affects a small subset of the genome (29). Evidence provided by DNA microarrays using malignant cell lines cultured with a HDAC inhibitor shows that there are a finite (1-2%) number of genes whose products are altered. For example, cells treated in culture with HDAC inhibitors show a consistent induction of the cyclin-dependent kinase inhibitor p21 (30). This protein plays an important role in cell cycle arrest. HDAC inhibitors are thought to increase the rate of transcription of p21 by propagating the hyperacetylated state of histones in the region of the p21 gene, thereby making the

gene accessible to transcriptional machinery. Genes whose expression is not affected by HDAC inhibitors do not display changes in the acetylation of regional associated histones (31).

## SUMMARY OF THE INVENTION

The present invention provides pharmaceutical compositions of an HDAC inhibitor and a chelatable metal compound. In one embodiment, the invention provides a method of treating cancer and alleviating the side effects of the HDAC inhibitor by administering the pharmaceutical composition. In another embodiment, the present invention also provides pharmaceutical compositions of metal HDAC inhibitor chelate complexes. In another embodiment, the invention provides methods of treating cancer by administering the pharmaceutical compositions. The invention provides crystalline compositions of metal HDAC inhibitor chelate complexes and methods of producing same.

## BRIEF DESCRIPTION OF THE DRAWINGS

**FIG. 1** Figure 1 shows the dissolution profile of SAHA from the reference capsule lot. The capsules contain about 100 mg of active ingredient SAHA and excipients.

**FIG. 2** Figure 2 shows the x-ray diffractogram for the crystalline iron SAHA chelate complex.

**FIG. 3** Figure 3 shows x-ray diffractograms for SAHA measured with Siemans D500 Automated Powder Diffractometer. Fig 3A-E: SAHA Form I-V.

**FIG. 4** Figure 4 shows x-ray diffractograms for SAHA Form I measured with X'PERT Pro Phillips X-ray diffractometer.

## DETAILED DESCRIPTION OF THE INVENTION

The term "pharmaceutically acceptable carrier" is intended to include any and all solvents, dispersion media, coatings, antibacterial and antifungal agents, isotonic and absorption delaying agents, and the like, compatible with pharmaceutical administration. Suitable carriers are described in the most recent edition of Remington's Pharmaceutical Sciences, a standard reference text in the field, which is incorporated herein by reference. Liposomes and non-aqueous vehicles such as fixed oils may also be used. The use of such media and agents for pharmaceutically active substances is well known in the art. Except insofar as any conventional media or agent is incompatible with the Compositions of this invention, use thereof in the compositions is contemplated. Supplementary active compounds can also be incorporated into the compositions.

The term "f2" or "F2" refers to a similarity factor determined through a point by point comparison of a new *in vitro* dissolution profile to a reference *in vitro* dissolution profile, as shown in equation 1.

$$f_2 = 50\log\left\{\left[1+1/n\sum_{t=1}^{n}(R_t - T_t)^2\right]^{-0.5} x\,100\right\} \qquad \text{(Equation 1)}$$

$R_t$ refers to the percent of compound dissolved at each time point (t) for the reference. $T_t$ refers to the percent of compound dissolved at each time point (t) for the test sample. *n* refers to the number of time points used for the calculation. $f_2$ values of 50 or greater are considered to reflect similar *in vitro* dissolution rates.

For the purpose of this invention, dissolution rates or profiles *in vitro* of the entire SAHA of the pharmaceutical composition is measured from the entire pharmaceutical composition according to the steps and conditions in Example 4. In one embodiment, dissolution rates or profiles *in vitro* is measured by using a USP Dissolution Apparatus II with a helical sinker (Quality Lab Accessories L.L.C., Manville, NJ) in 900 mL of 2.0% Tween (TCI America, Portland, Oregon) at a temperature of 37±0.5°C, and paddles rotated at 100 rpm. The entire pharmaceutical composition includes the entire SAHA and the chelatable metal compound and if the pharmaceutical composition contains a capsule shell, carrier, excipient, diluent, disintegrating agent, lubricant, binder or any additional agent described in the Pharmaceutical Composition Section below, the measurement is performed with those components.

The term "about" when used in the context of an amount refers to ±10% of the specified amount.

For the purpose of this invention, for X-ray diffraction patterns, depending on the calibration, sample or instrumentation, peaks at 2θ can shift up to ±0.3 degrees (error) in one direction. For example, all peaks in X-ray diffraction pattern shift up to +0.3 degrees, or up to - 0.3 degrees. An X-ray diffraction pattern or peaks within that error is considered the same or substantially similar.

Pharmaceutical Composition comprising HDAC Inhibitor and Chelatable metal compound

In one embodiment, the invention provides a pharmaceutical composition comprising a therapeutically effective amount of Histone Deacetylase (HDAC) inhibitor and a chelatable metal compound, and a pharmaceutically acceptable carrier. The pharmaceutical composition can form the metal HDAC inhibitor chelate complex in vivo when administered to the subject.

In one embodiment, the HDAC inhibitor is a hydroxamic acid derivative. In another embodiment, the HDAC inhibitor has a metal-binding (i.e. iron or zinc) ligand, for example carbonyl group, hydroxyl group, amino group, thiol group, amide group, and other groups found in known HDAC inhibitors (eg. Benzamide). In one embodiment, the HDAC inhibitor is suberoylanilide hydroxamic acid (SAHA) or pharmaceutically acceptable salt or hydrate or solvate thereof. In another embodiment, the entire SAHA in the pharmaceutical composition has an in vitro dissolution profile with a similarity factor (f2) of at least 50 to 100 compared to the reference dissolution profile shown in Figure 1. In one embodiment, f2 is 56 to 100. In one embodiment, f2 is 60 to 100. In one embodiment, f2 is 65 to 100. In another embodiment, f2 is 80 to 100. In a further embodiment, the pharmaceutical composition is a single capsule, wherein the amount of the SAHA is about 100 mg.

In one embodiment, the entire SAHA in the pharmaceutical composition is 43-63% dissolved at 10 minutes, 66-86% dissolved at 30 minutes, and 77-97% dissolved at 60 minutes *in vitro.* In one embodiment, the entire SAHA of the pharmaceutical composition is 52-72% dissolved at 15 minutes, 66-86% dissolved at 30 minutes, and 73-93% dissolved at 45 minutes *in vitro.* In another embodiment, the entire SAHA of the pharmaceutical composition is 43-63% dissolved at 10 minutes, 52-72% dissolved at 15 minutes, 58-78% dissolved at 20 minutes, 66-86% dissolved at 30 minutes, 73-93% dissolved at 45 minutes and 77-97% dissolved at 60 minutes *in vitro.* In one embodiment, the entire SAHA of the pharmaceutical composition is 46-60% dissolved at 10 minutes, 55-69% dissolved at 15 minutes, 61-75% dissolved at 20 minutes, 69-83% dissolved at 30 minutes, 76-90% dissolved at 45 minutes, and 80-94% dissolved at 60 minutes *in vitro.* In one embodiment, at least 45% but less than or equal to 75% of the entire SAHA is dissolved at 15 minutes, at least 75% of the entire SAHA is dissolved in 60 minutes.

The HDAC inhibitor or chelatable metal compound can be in amorphous form. The HDAC inhibitor or chelatable metal compound may be micronized, or may be agglomerated, particulate granules, powders, oils, oily suspensions or any other form of solid.

In a particular embodiment, the HDAC inhibitor is crystalline suberoylanilide hydroxamic acid. In one embodiment, crystalline SAHA is SAHA Form I and characterized by an X-ray diffraction pattern substantially similar to that set forth in Figure 3A. In another embodiment, crystalline SAHA is SAHA Form I and characterized by an X-ray diffraction pattern substantially similar to that set forth in Figure 4. In one embodiment, SAHA Form I is characterized by an X-ray powder diffraction pattern with copper K$\alpha$ radiation, including characteristic peaks at 9.0, 9.4, 17.5, 19.4, 20.0, 24.0, 24.4, 24.8, 25.0, 28.0 degrees 2$\theta$. In another embodiment, SAHA Form I is characterized by an X-ray powder diffraction pattern with copper K$\alpha$ radiation, including characteristic peaks at about 9.0, 9.4, 17.5, 19.4, 20.0, 24.0, 24.4, 24.8, 25.0, 28.0, and 43.3 degrees 2$\theta$. In one embodiment, the SAHA Form I is characterized by an X-ray diffraction powder pattern with copper K$\alpha$ radiation, including characteristic peaks at 9.4, 17.5, 19.4, 20.0, 24.0, and 28.0 degrees 2$\theta$. In another embodiment, suberoylanilide hydroxamic acid (SAHA) Form I is characterized by an X-ray powder diffraction pattern including characteristic peaks at about 9.4, 17.5, 19.4, 20.0, 24.0, and 28.0 degrees 2$\theta$,. wherein the X-ray powder diffraction is measured with a Copper X-ray source; and further characterized by a Differential Scanning Calorimetry (DSC) thermogram having a single maximum value at about 164.4$\pm$2.0, as measured by a Perkins Elmer DSC 6 Instrument. In another embodiment, suberoylanilide hydroxamic acid (SAHA) Form I is characterized by an X-ray powder diffraction pattern including characteristic peaks at about 9.4, 17.5, 19.4, 20.0, 24.0, and 28.0 degrees 2$\theta$, and lacking peaks at about 13.4-14.0 and 22.7-23.0 degrees 2$\theta$,. wherein the X-ray powder diffraction is measured with a Copper X-ray source. In a further embodiment, suberoylanilide hydroxamic acid Form I is characterized by an X-ray powder diffraction pattern including characteristic peaks at 9.1, 10.8, 12.3, 17.2, 19.2, 19.8, 23.7, 24.1, 25.7, 26.8 and 27.7 degrees 2$\theta$ and lacking peaks at 13.4-14.0 and 22.7-23.0 degrees 2$\theta$ using a Copper X-ray source.

In one embodiment, the SAHA Form I is characterized by an X-ray powder diffraction pattern with copper K$\alpha$ radiation, including characteristic peaks at 9.0, 9.4, 17.5, 19.4, 20.0, 24.0, 24.4, 24.8, 25.0, 28.0 degrees 2$\theta$, and lacking peaks at 13.4-14.0 and 22.7-23.0 degrees 2$\theta$. In another embodiment, SAHA Form I is characterized by an X-ray powder diffraction pattern with copper K$\alpha$ radiation, including characteristic peaks at 9.0, 9.4, 17.5, 19.4, 20.0, 24.0, 24.4, 24.8, 25.0, 28.0, 43.3 degrees 2$\theta$ and lacking peaks at 13.4-14.0 and 22.7-23.0 degrees 2$\theta$. In a further embodiment, SAHA Form I is additionally characterized by the lack of at least one peak at about <8.7, 10.0-10.2, 13.4-14.0, 15.0-15.2, 17.5-19.0, 20.1-20.3, 21.1-21.3, 22.0.-22.22, 22.7-23.0, 25.0-25.5, 26.0-26.2, and 27.4-27.6 degrees 2$\theta$.

In another embodiment, SAHA Form I is further characterized by a Differential Scanning Calorimetry (DSC) thermogram having a single maximum value at about 164.4$\pm$2.0, as measured by a Perkins Elmer DSC 6 Instrument. In one embodiment, SAHA Form I has unit cell parameters of a= 10.9 Å, b= 7.9 Å, c= 16.4 Å, $\alpha$= 90°, $\beta$= 97.8°, $\gamma$=90°, space group $P2_1/n$.

In a particular embodiment, the crystalline suberoylanilide hydroxamic acid is SAHA Form IV and is characterized by an X-ray powder diffraction pattern with copper Kα radiation, including characteristic peaks at about 8.8, 9.3, 11.0, 12.4, 17.4, 19.4, 19.9, 22.4, 22.9, 23.83, 24.2, 24.8, 25.8, 27.0, 27.8, 28.4 degrees 2θ.

In one embodiment, the invention provides a single capsule comprising about 100 mg suberoylanilide hydroxamic acid, and the chelatable metal compound, wherein the entire SAHA has an in vitro dissolution profile characterized by: at least 45% but less than or equal to 75% of the entire SAHA is dissolved at 15 minutes, at least 75% of the entire SAHA is dissolved in 60 minutes, wherein the SAHA is crystalline and characterized by an X-ray powder diffraction pattern with copper Kα radiation, including characteristic peaks at 9.0, 9.4, 17.5, 19.4, 20.0, 24.0, 24.4, 24.8, 25.0, 28.0 degrees 2θ, and lacking peaks at 13.4-14.0 and 22.7-23.0 degrees 2θ.

In one embodiment, the invention provides a single capsule comprising about 100 mg suberoylanilide hydroxamic acid, and the chelatable metal compound, wherein the entire SAHA has an in vitro dissolution profile characterized by: at least 45% but less than or equal to 75% of the entire SAHA is dissolved at 15 minutes, at least 75% of the entire SAHA is dissolved in 60 minutes, wherein the SAHA is crystalline and characterized by an X-ray powder diffraction pattern with copper Kα radiation, including characteristic peaks at 9.4, 17.5, 19.4, 20.0, 24.0, and 28.0 degrees 20,. wherein the X-ray diffraction is measured with a Copper X-ray source; and further characterized by a Differential Scanning Calorimetry (DSC) thermogram having a single maximum value at about 164.4±2.0, as measured by a Perkins Elmer DSC 6 Instrument.

In one embodiment, the invention provides a single capsule comprising about 100 mg suberoylanilide hydroxamic acid, and the chelatable metal compound, wherein the entire SAHA has an in vitro dissolution profile characterized by: at least 45% but less than or equal to 75% of the entire SAHA is dissolved at 15 minutes, at least 75% of the entire SAHA is dissolved in 60 minutes, wherein the SAHA is crystalline and characterized by an X-ray powder diffraction pattern with copper Kα radiation, including characteristic peaks at 9.1, 10.8, 12.3, 17.2, 19.2, 19.8, 23.7, 24.1, 25.7, 26.8 and 27.7 degrees 2θ and lacking peaks at 13.4-14.0 and 22.7-23.0 degrees 2θ using a Copper X-ray source.

In another embodiment, the invention provides a single capsule comprising about 100 mg suberoylanilide hydroxamic acid, and the chelatable metal compound, wherein the entire SAHA has an in vitro dissolution profile with a similarity factor (f2) of at least 50 to 100 compared to the reference dissolution profile shown in Figure 1, wherein the SAHA is crystalline and characterized by an X-ray powder diffraction pattern with copper Kα radiation, including characteristic peaks at 9.0, 9.4, 17.5, 19.4, 20.0, 24.0, 24.4, 24.8, 25.0, 28.0 degrees 2θ, and lacking a peak at 13.4-14.0 and 22.7-23.0 degrees 2θ.

In another embodiment, the invention provides a single capsule comprising about 100 mg suberoylanilide hydroxamic acid, and the chelatable metal compound, wherein the entire SAHA has an in vitro dissolution profile with a similarity factor (f2) of at least 50 to 100 compared to the reference dissolution profile shown in Figure 1, wherein the SAHA is crystalline and characterized by an X-ray powder diffraction pattern with copper Kα radiation, including characteristic peaks at 9.4, 17.5, 19.4, 20.0, 24.0, and 28.0 degrees 2θ,. wherein the X-ray diffraction is measured with a Copper X-ray source; and further characterized by a Differential Scanning Calorimetry (DSC) thermogram having a single maximum value at about 164.4±2.0, as measured by a Perkins Elmer DSC 6 Instrument.

In another embodiment, the invention provides a single capsule comprising about 100 mg suberoylanilide hydroxamic acid, and the chelatable metal compound, wherein the entire SAHA has an in vitro dissolution profile with a similarity factor (f2) of at least 50 to 100 compared to the reference dissolution profile shown in Figure 1, wherein the SAHA is crystalline and characterized by an X-ray powder diffraction pattern with copper Kα radiation, including characteristic peaks at 9.1, 10.8, 12.3, 17.2, 19.2, 19.8, 23.7, 24.1, 25.7, 26.8 and 27.7 degrees 2θ and lacking peaks at 13.4-14.0 and 22.7-23.0 degrees 2θ using a Copper X-ray source.

In a further embodiment, the invention provides a single capsule comprising about 100 mg suberoylanilide hydroxamic acid, and the chelatable metal compound, wherein the entire SAHA has an in vitro dissolution profile characterized by 43-63% dissolved at 10 minutes, 66-86% dissolved at 30 minutes, and 77-97% dissolved at 60 minutes, wherein the SAHA is crystalline and characterized by an X-ray powder diffraction pattern with copper Kα radiation, including characteristic peaks at 9.0, 9.4, 17.5, 19.4, 20.0, 24.0, 24.4, 24.8, 25.0, 28.0 degrees 2θ, and lacking a peak at 13.4-14.0 and 22.7-23.0 degrees 2θ.

In a further embodiment, the invention provides a single capsule comprising about 100 mg suberoylanilide hydroxamic acid, and the chelatable metal compound, wherein the entire SAHA has an in vitro dissolution profile characterized by 43-63% dissolved at 10 minutes, 66-86% dissolved at 30 minutes, and 77-97% dissolved at 60 minutes, wherein the SAHA is crystalline and characterized by an X-ray powder diffraction pattern with copper Kα radiation, including characteristic peaks at 9.4, 17.5, 19.4, 20.0, 24.0, and 28.0 degrees 2θ,. wherein the X-ray diffraction is measured with a Copper X-ray source; and further characterized by a Differential Scanning Calorimetry (DSC) thermogram having a single maximum value at about 164.4±2.0, as measured by a Perkins Elmer DSC 6 Instrument.

In a further embodiment, the invention provides a single capsule comprising about 100 mg suberoylanilide hydroxamic acid, and the chelatable metal compound, wherein the entire SAHA has an in vitro dissolution profile characterized by 43-63% dissolved at 10 minutes, 66-86% dissolved at 30 minutes, and 77-97% dissolved at 60 minutes,

wherein the SAHA is crystalline and characterized by an X-ray powder diffraction pattern with copper Kα radiation, including characteristic peaks at 9.1, 10.8, 12.3, 17.2, 19.2, 19.8, 23.7, 24.1, 25.7, 26.8 and 27.7 degrees 2θ and lacking peaks at 13.4-14.0 and 22.7-23.0 degrees 2θ using a Copper X-ray source.

In a further embodiment, the invention provides a single capsule comprising about 100 mg suberoylanilide hydroxamic acid, and the chelatable metal compound, wherein the entire SAHA has an in vitro dissolution profile characterized by 43-63% dissolved at 10 minutes, 66-86% dissolved at 30 minutes, and 77-97% dissolved at 60 minutes, wherein the SAHA is crystalline and characterized by an X-ray powder diffraction pattern with copper Kα radiation, including characteristic peaks at 9.0, 9.4, 17.5, 19.4, 20.0, 24.0, 24.4, 24.8, 25.0, 28.0 degrees 2θ, and lacking peaks at 13.4-14.0 and 22.7-23.0 degrees 2θ.

In one embodiment, the chelatable metal compound comprises iron. The iron compound can be in any form bioavailable to the patient that forms the iron HDAC inhibitor chelate complex in situ, for example, a pharmaceutical composition form such as tablet or capsule form. In one embodiment, the iron compound is an iron supplement in the form of ferrous gluconate, ferric gluconate, ferrous sulfate, ferric sulfate, ferrous fumarate, ferric fumarate, iron amino acid chelate or ferrous bis-glycinate. The iron supplement may also contain other vitamins and minerals. In another embodiment, the HDAC inhibitor and iron in the pharmaceutical composition is at a 5:1, 4:1, 3:1, 2:1, 1:1, or 2:1 stoichiometric ratio. In one embodiment, the pharmaceutical composition comprises about 100 mg of SAHA and about 1 to 10 molar equivalents of iron supplement. In another embodiment, the pharmaceutical composition comprises about 150 mg of SAHA and about 1 to 10 molar equivalents of iron supplement. In a further embodiment, the pharmaceutical composition comprises about 200 mg of SAHA and about 1 to 10 molar equivalents of iron supplement. In an alternative embodiment, the pharmaceutical composition comprises about 50 mg of SAHA and about 1 to 10 molar equivalents of iron supplement. In one embodiment, the chelatable metal compound comprises zinc. The zinc compound can be in any form bioavailable to the patient and that forms the zinc HDAC inhibitor chelate complex in situ, for example, in pharmaceutical composition form such as tablet or capsule form. In one embodiment, the zinc compound is a zinc supplement in the form of zinc gluconate, zinc picolinate, zinc citrate, zinc amino acid chelate or zinc oxide. The zinc supplement may also contain other vitamins and minerals. In another embodiment, the SAHA and zinc in the pharmaceutical composition is at a 5:1, 4:1, 3:1, 2:1, 1:1, or 2:1 stoichiometric ratio of SAHA to zinc. In one embodiment, the pharmaceutical composition comprises about 100 mg of SAHA and about 1 to 10 molar equivalents of zinc supplement. In another embodiment, the pharmaceutical composition comprises about 150 mg of SAHA and about 1 to 10 molar equivalents of zinc supplement. In a further embodiment, the pharmaceutical composition comprises about 200 mg of SAHA and about 1 to 10 molar equivalents of zinc supplement.

Pharmaceutical Compositions comprising Metal HDAC inhibitor Chelate Complex

In one embodiment, the invention provides a pharmaceutical composition comprising a therapeutically effective amount of metal HDAC inhibitor chelate complex or hydrate or solvate thereof and a pharmaceutically acceptable carrier.

The metal HDAC inhibitor chelate complex can be in amorphous form. The metal HDAC inhibitor complex may be crystalline, micronized, or may be agglomerated, particulate granules, powders, oils, oily suspensions or any other form of solid. The metal HDAC inhibitor chelate complex or hydrate or solvate thereof can be in any crystalline form. In one embodiment, the HDAC inhibitor is a hydroxamic acid derivative. In one embodiment, the HDAC inhibitor is SAHA.

In one embodiment, the invention provides a pharmaceutical composition comprising a therapeutically effective amount of iron SAHA chelate complex or hydrate or solvate thereof and a pharmaceutically acceptable carrier. In one embodiment, the iron to SAHA ratio is 1:3, 1:2 or 1:1.

In one embodiment, the iron SAHA chelate complex is crystalline, and is characterized by an X-ray diffraction pattern substantially similar to that set forth in Figure 1. In another embodiment, the iron SAHA chelate complex is characterized by an X-ray powder diffraction pattern with copper Kα radiation, including characteristic peaks at 8.8, 14.5 and 21.8 degrees 2θ. In a further embodiment, the iron SAHA chelate complex is characterized by an X-ray powder diffraction pattern with copper Kα radiation, including characteristic peaks at 8.8, 13.3, 14.5, 20.3, 21.8 and 24.6 degrees 2θ. In a further embodiment, the iron SAHA chelate complex is characterized by an X-ray powder diffraction pattern with copper Kα radiation, including characteristic peaks at 8.8, 13.3, 14.5, 18.5, 20.3, 21.8, 24.6, 25.8 and 33.3 degrees 2θ.

In another embodiment, the invention also provides a pharmaceutical composition comprising a therapeutically effective amount of zinc SAHA chelate complex or hydrate or solvate thereof and a pharmaceutically acceptable carrier. In one embodiment, the zinc to SAHA ratio is 1:3, 1:2 or 1:1.

The invention also encompasses pharmaceutical compositions comprising hydrates or solvates of the metal HDAC inhibitor chelate complex or SAHA. The term "hydrate" includes but is not limited to hemihydrate, monohydrate, dihydrate, trihydrate and the like.

Pharmaceutical Compositions

The pharmaceutically accetaptable carrier in the pharmaceutical compositions can be in solid particle form. Any inert excipient that is commonly used as a carrier or diluent may be used in the formulations of the present invention, such as for example, a gum, a starch, a sugar, a cellulosic material, an acrylate, or mixtures thereof. In one embodiment, the diluent is microcrystalline cellulose. The Compositions of the present invention (for example, HDAC inhibitor; chelatable metal compound; HDAC inhibitor and chelatable metal compound; or metal HDAC inhibitor chelate complex) may further comprise a disintegrating agent (e.g., croscarmellose sodium) and a lubricant (e.g., magnesium stearate), and in addition may comprise one or more additives selected from a binder, a buffer, a protease inhibitor, a surfactant, a solubilizing agent, a plasticizer, an emulsifier, a stabilizing agent, a viscosity increasing agent, a sweetener, a film forming agent, or any combination thereof. Furthermore, the Compositions of the present invention may be in the form of controlled release or immediate release formulations.

In one embodiment, the pharmaceutical composition described herein may further be comprised of microcrystalline cellulose, croscarmellose sodium and magnesium stearate. The percentage of the Compositions of this invention and various excipients in the formulations may vary. For example, the pharmaceutical composition may comprise between about 20 and 90%, between about 50-80% or between about 60-70% by weight of the Compositions of this invention. Furthermore, the pharmaceutical composition may comprise between about about 10 and 70%, between about 20-40%, between about 25-35% by weight microcrystalline cellulose as a carrier or diluent. Furthermore, the pharmaceutical composition may comprise between about 1 and 30%, between about 1-10%, between about 2-5% by weight croscarmellose sodium as a disintegrant. Furthermore, the pharmaceutical composition may comprise between about 0.1-5% or about 0.5-1.5% by weight magnesium stearate as a lubricant.

In one embodiment, the pharmaceutical composition of the invention is about 50-80% by weight of Compositions of this invention; about 20-40% by weight microcrystalline cellulose; about 1-10% by weight croscarmellose sodium; and about 0.1-5% by weight magnesium stearate. In another embodiment, the pharmaceutical composition of the invention is about 60-70% by weight of Compositions of this invention; about 25-35% by weight microcrystalline cellulose; about 2-5% by weight croscarmellose sodium; and about 0.5-1.5% by weight magnesium stearate. In one embodiment, the pharmaceutical composition described comprises about 50-200 mg or 50-600 mg of SAHA Form I.

A particular embodiment of the invention is a solid formulation of the Compositions of this invention with microcrystalline cellulose, NF (Avicel Ph 101), sodium croscarmellose, NF (AC-Di-Sol) and magnesium stearate, NF, contained in a gelatin capsule. A further embodiment is a pharmaceutical composition comprising about 100 mg Compositions of this invention, about 44.3 mg of microcrystalline cellulose, about 4.5 mg of croscarmellose sodium, about 1.2 mg of magnesium stearate.

In one embodiment, the pharmaceutical compositions are administered orally, and are thus formulated in a form suitable for oral administration, i.e., as a solid or liquid form. Suitable solid oral formulations include for example, tablets, capsules, pills, granules, pellets and the like. Suitable liquid oral formulations include for example, emulsions, oils and the like. In one embodiment of the present invention, the Composition is formulated in a capsule. In accordance with this embodiment, the pharmaceutical compositions of the present invention comprise a hard gelatin capsule in addition to the Compositions of this invention and the inert carrier or diluent.

Solid carriers/diluents include, but are not limited to, a gum, a starch (e.g., corn starch, pregelatinized starch), a sugar (e.g., lactose, mannitol, sucrose, dextrose), a cellulosic material (e.g., microcrystalline cellulose), an acrylate (e.g., polymethylacrylate), calcium carbonate, magnesium oxide, talc, or mixtures thereof.

For liquid formulations, pharmaceutically acceptable carriers may be non-aqueous solutions, suspensions, emulsions or oils. Examples of non-aqueous solvents are propylene glycol, polyethylene glycol, and injectable organic esters such as ethyl oleate. Examples of oils are those of petroleum, animal, vegetable, or synthetic origin, for example, peanut oil, soybean oil, mineral oil, olive oil, sunflower oil, and fish-liver oil. Suspensions can also include the following components: fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid (EDTA).

In addition, the pharmaceutical compositions may further comprise binders (e.g., acacia, cornstarch, gelatin, carbomer, ethyl cellulose, guar gum, hydroxypropyl cellulose, hydroxypropyl methyl cellulose, povidone), disintegrating agents (e.g., cornstarch, potato starch, alginic acid, silicon dioxide, croscarmellose sodium, crospovidone, guar gum, sodium starch glycolate, Primogel), detergents (e.g., Tween 20, Tween 80, Pluronic F68, bile acid salts), protease inhibitors, surfactants (e.g., sodium lauryl sulfate), permeation enhancers, solubilizing agents (e.g., glycerol, polyethylene glycerol), a glidant (e.g., colloidal silicon dioxide), antioxidants (e.g., ascorbic acid, sodium metabisulfite, butylated hydroxyanisole), stabilizers (e.g., hydroxypropyl cellulose, hyroxypropylmethyl cellulose), viscosity increasing agents (e.g., carbomer, colloidal silicon dioxide, ethyl cellulose, guar gum), sweeteners (e.g., sucrose, aspartame, citric acid), flavoring agents (e.g., peppermint, methyl salicylate, or orange flavoring), preservatives (e.g., Thimerosal, benzyl alcohol, parabens), lubricants (e.g., stearic acid, magnesium stearate, polyethylene glycol, sodium lauryl sulfate), flow-aids (e.g.,

colloidal silicon dioxide), plasticizers (e.g., diethyl phthalate, triethyl citrate), emulsifiers (e.g., carbomer, hydroxypropyl cellulose, sodium lauryl sulfate), polymer coatings (e.g., poloxamers or poloxamines), coating and film forming agents (e.g., ethyl cellulose, acrylates, polymethacrylates) and/or adjuvants.

In one embodiment, the Compositions of this invention is prepared with carriers that will protect the Compositions against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Methods for preparation of such formulations will be apparent to those skilled in the art. The materials can also be obtained commercially from Alza Corporation and Nova Pharmaceuticals, Inc. Liposomal suspensions (including liposomes targeted to infected cells with monoclonal antibodies to viral antigens) can also be used as pharmaceutically acceptable carriers. These can be prepared according to methods known to those skilled in the art, for example, as described in U.S. Patent No. 4,522,811.

The preparation of pharmaceutical compositions that contain an active component is well understood in the art, for example, by mixing, granulating, or tablet-forming processes. For oral administration, the active agents are mixed with additives customary for this purpose, such as vehicles, stabilizers, or inert diluents, and converted by customary methods into suitable forms for administration, such as tablets, coated tablets, hard or soft gelatin capsules, aqueous, alcoholic or oily solutions and the like as detailed above.

In one embodiment, the oral compositions are formulated in dosage unit form for ease of administration and uniformity of dosage. Dosage unit form as used herein refers to physically discrete units suited as unitary dosages for the subject to be treated; each unit containing a predetermined quantity of Compositions of this invention calculated to produce the desired therapeutic effect in association with the required pharmaceutical carrier. The specification for the dosage unit forms of the invention are dictated by and directly dependent on the unique characteristics of the Compositions of this invention and the particular therapeutic effect to be achieved, and the limitations inherent in the art of compounding such an active compound for the treatment of individuals. In certain embodiments, the dosage unit contains about 600 mg, 550 mg, 500 mg, 450 mg, 400 mg, 350 mg, 300 mg, 250 mg, 200 mg, 150 mg, 110 mg, 105 mg, 100 mg, 95 mg, 90 mg, 85 mg, 80 mg, 75 mg, 70 mg, 65 mg, 60 mg, 55 mg, 50 mg, 45 mg, or 40 mg of Compositions of this invention. In one embodiment, the amount of the Compositions of this invention is about 100 mg.

The pharmaceutical compositions can be included in a container, pack, or dispenser together with instructions for administration. In one embodiment, the pharmaceutical composition is a single capsule, wherein the amount of the Compositions of this invention is about 100 mg. In one embodiment, the pharmaceutical composition is two capsules, wherein each capsule contains Compositions of this invention of about 50 mg.

## Method of Obtaining a Metal HDAC Inhibitor Chelate Complex and Crystallization

The invention also provides a method of obtaining a metal HDAC inhibitor chelate complex comprising the step of adding a chelatable metal compound and base with the HDAC inhibitor in a reaction medium. In one embodiment, the reaction medium is an organic solvent or mixture of organic solvent and water. In one embodiment, the organic solvent is ethanol. In one embodiment, the chelatable metal compound comprises iron or zinc. In another embodiment, the chelatable metal compound, base and HDAC inhibitor are soluble in the reaction medium. In one embodiment, the base is N,N-diisopropylethylamine or sodium ethoxide. In one embodiment, the chelatable metal compound is ferric chloride or zinc chloride.

The invention also provides a method of obtaining a crystalline metal HDAC inhibitor chelate complex comprising the step of crystallizing the chelate complex in an organic solvent or in a mixture of organic solvent and water. In one embodiment, the metal is zinc or iron.

In one particular embodiment, the crystalline metal HDAC inhibitor chelate complex is crystallized from an organic solvent. The organic solvent may be an alcohol such as methanol, ethanol or isopropanol. In one embodiment, the organic solvent is one or more of methanol, ethanol, acetonitrile, isopropanol and acetic acid. In one embodiment, the organic solvent is ethanol. In a particular embodiment, the organic solvent used in the reaction medium is the same as that in the crystallization.

In another embodiment, the mixture of organic solvent and water comprises about 1-99% organic solvent and about 99-1% of water. In another embodiment, the mixture comprises 40-99% ethanol and 60%-1% of water. In one embodiment, the mixture comprises about 15-85% organic solvent and about 1-15% water. In a particular embodiment, the mixture comprises about 85% organic solvent and about 15% water. In another particular embodiment, the mixture comprises 1:1 ethanol and water. In yet another particular embodiment, the mixture comprises 9:1 ethanol and water. The ratios or percentages of organic solvent to water described here are by volume.

In one particular embodiment, the organic solvent is an alcohol (e.g. methanol, ethanol, isopropanol and the like). However, it should be apparent to a person skilled in the art that the crystallizations or reactions described herein can be carried out in any suitable solvents or solvent mixtures which may be readily selected by one of skill in the art of organic synthesis. Such suitable organic solvents, as used herein may include, by way of example and without limitation,

chlorinated solvents, hydrocarbon solvents, ether solvents, polar protic solvents and polar aprotic solvents. Suitable halogenated solvents include, but are not limited to carbon tetrachloride, bromodichloromethane, dibromochloromethane, bromoform, chloroform, bromochloromethane, dibromomethane, butyl chloride, dichloromethane, tetrachloroethylene, trichloroethylene, 1,1,1-trichloroethane, 1,1,2-trichloroethane, 1,1-dichloroethane, 1,2-dichloroethane, 2-chloropropane, hexafluorobenzene, 1,2,4-trichlorobenzene, o-dichlorobenzene, chlorobenzene, fluorobenzene, fluorotrichloromethane, chlorotrifluoromethane, bromotrifluoromethane, carbon tetrafluoride, dichlorofluoromethane, chlorodifluoromethane, trifluoromethane, 1,2-dichlorotetrafluorethane and hexafluoroethane. Suitable hydrocarbon solvents include, but are not limited to benzene, cyclohexane, pentane, hexane, toluene, cycloheptane, methylcyclohexane, heptane, ethylbenzene, m-, o-, or p-xylene, octane, indane, nonane. Suitable ether solvents include, but are not limited to dimethoxymethane, tetrahydrofuran, 1,3-dioxane, 1,4-dioxane, furan, diethyl ether, ethylene glycol dimethyl ether, ethylene glycol diethyl ether, diethylene glycol dimethyl ether, diethylene glycol diethyl ether, triethylene glycol diisopropyl ether, anisole, or t-butyl methyl ether.

Suitable polar protic solvents include, but are not limited to methanol, ethanol, 2-nitroethanol, 2-fluoroethanol, 2,2,2-trifluoroethanol, ethylene glycol, 1-propanol, 2-propanol, 2-methoxyethanol, 1-butanol, 2-butanol, i-butyl alcohol, t-butyl alcohol, 2-ethoxyethanol, diethylene glycol, 1-, 2-, or 3- pentanol, neo-pentyl alcohol, t-pentyl alcohol, diethylene glycol monomethyl ether, diethylene glycol monoethyl ether, cyclohexanol, benzyl alcohol, phenol, and glycerol. Suitable polar aprotic solvents include, but are not limited to dimethylformamide (DMF), dimethylacetamide (DMAC), 1,3-dimethyl-3,4,5,6-tetrahydro-2(1H)-pyrimidinone (DMPU), 1,3-dimethyl-2-imidazolidinone (DMI), N- methylpyrrolidinone (NMP), formamide, N-methylacetamide, N-methylformamide, acetonitrile (ACN), dimethylsulfoxide, propionitrile, ethyl formate, methyl acetate, hexachloroacetone, acetone, ethyl methyl ketone, ethyl acetate, isopropyl acetate, t-butyl acetate, sulfolane, N,N-dimethylpropionamide, nitromethane, nitrobenzene, hexamethylphosphoramide.

Methods of Treatment

HDAC inhibitors that possess metal binding domains, such as the hydroxamic acid derivatives, are capable of sequestering certain endogenous salts, such as iron and zinc. These salts are required by biological systems for long-term health and survival. Additionally, chelatable salts such as iron are required by enzymes and proteins such as: lipoxygenases, the P-450 superfamily of enzymes, monoamine oxidases, heme and numerous others, to maintain normal function.

Zinc is essential for cell division and growth and assists in the formation of DNA. Zinc is also important for assisting in the proper functioning of insulin and is involved in immune system health. Zinc deficiency can cause intestinal mucosal atrophy, anemia, diarrhea, decreased water and nutrient absorption (dehydration and anorexia), impaired taste, alopecia, thymic atrophy, testes atrophy; hyperglycemia, increased serum glucose, creatine increase, renal insufficiency, impairment or failure.

Clinical adverse experiences or side effects such as diarrhea, fatigue, nausea, thrombocytopenia, anorexia, dysgeusia, weight decrease, muscle spasms, alopecia, anemia, blood creatinine increase, vomiting, chills have been observed for SAHA in the treatment of patients with cancer, some of which may be associated with the deficiency of iron or zinc as a result of the sequestering of these ions by the metal binding domain of SAHA. Preclinical toxicity of SAHA include weight loss or inappetence, immune suppression (leucopenia, lymphopenia, thymic atrophy) in all animal species; thrombocytopenia in rats; gastrointestinal toxicity (mucosal atrophy, acute inflammation, necrosis, multiple lesions), dehydration, electrolyte loss, testicular atrophy in dogs.

Likewise, other zinc chelators share similar toxicities or side effects as HDAC inhibitors. For example, Lithostat (acetohydroxamic acid) causes side effects such as nausea, vomiting, anorexia, fatigue, malaise, reticulocytosis, anemia, thrombocytopenia, leukopenia and headache. Desferrioxamine (desferiprone) causes side effects such as fatigue, bone marrow atrophy, thymic atrophy and neutropenia.

Thus, the co-administration/co-formulation of HDAC inhibitors and a chelatable metal compound, such as iron or zinc, would be beneficial in maintaining the availability of iron, zinc and other chelatable salt stores, and eliminate the side effects of HDAC inhibitors. In addition to co-administration and co-formulation, it is envisioned that the pre-formation of the metal HDAC inhibitor chelate complex would have a similar effect.

Thus, in one embodiment, the invention provides a method of treating cancer and alleviating the side effects of the HDAC inibitor comprising the step of co-administering to a patient a therapeutically effective amount of HDAC inhibitor and a chelatable metal compound. In another embodiment, the invention also provides a use of an HDAC inhibitor for the preparation of a medicament for the treatment of cancer and alleviating the side effects of the HDAC inibitor comprising the step of co-administering to a patient a therapeutically effective amount of HDAC inhibitor and a chelatable metal compound. In one embodiment, the chelatable metal compound is administered daily to the patient. In another embodiment, the chelatable metal compound is administered before or after administration of the HDAC inhibitor. In another embodiment, the chelatable metal compound is simultaneously administered with the HDAC inhibitor. In a further embodiment, the invention provides a kit comprising at least one pharmaceutically effective unit dosage of HDAC

inhibitor, and instructions for the treatment of cancer and alleviating the side effects of the HDAC inhibitor by co-administering a chelatable metal compound with the HDAC inhibitor. The pharmaceutically effective unit dosage of SAHA, pharmaceutically acceptable salt or hydrate thereof can be 200 mg, 300 mg, 400 mg, 500 mg or 600 mg. The chelatable metal compound can be in the form of a pharmaceutical composition, for example a vitamin supplement or mineral supplement, which can also comprise other vitamins and minerals.

In another embodiment, the invention also provides a method of treating cancer and alleviating the side effects of the HDAC inibitor comprising the step of administering to a patient a pharmaceutical composition comprising an HDAC inhibitor and a chelatable metal compound. In another embodiment, the invention provides a use for the preparation of a medicament comprising an HDAC inhibitor and a chelatable metal compound for the treatment of cancer and alleviating the side effects of the HDAC inhibitor. In a further embodiment, the invention provides a pharmaceutical composition comprising HDAC inhibitor and a chelatable metal compound, for use in treatment of cancer and to alleviate the side effects of the HDAC inhibitor. In one embodiment, the chelatable metal compound comprises iron or zinc. In another embodiment, the HDAC inhibitor is suberoylanilide hydroxamic acid (SAHA).

In one embodiment, the side effect is nausea, vomiting, diarrhea, anorexia, intestinal mucosal atrophy or gastrointestinal toxicity. In another embodiment, the side effect is anemia, fatigue or thrombocytopenia. In a further embodiment, the side effect is nausea, vomiting, diarrhea, anorexia, intestinal mucosal atrophy, gastrointestinal, fatigue or thrombocytopenia.

In a further embodiment, the invention further provides a method of treating cancer comprising the step of administering to a patient a therapeutically effective amount of metal HDAC inhibitor chelate complex or hydrate or solvate thereof. In another embodiment, the invention provides a use for the preparation of a medicament comprising a therapeutically effective amount of metal HDAC inhibitor chelate complex or hydrate or solvate thereof for the treatment of cancer. In a further embodiment, the invention provides a pharmaceutical composition comprising the metal HDAC inhibitor chelate complex or hydrate or solvate thereof, for use in treatment of cancer.

The invention further provides a method of treating cancer comprising the step of administering to a patient a therapeutically effective amount of the zinc or iron SAHA chelate complex or hydrate or solvate thereof mentioned in the foregoing embodiments.

In an embodiment, the method of the present invention is for the treatment of human patients with cancer. However, it is also likely that the method would be effective in the treatment of cancer in other mammals. Cancer includes but is not limited to any cancer caused by the proliferation of neoplastic cells, such as lung cancer, acute lymphoid myeloma, Hodgkins lymphoma, non-Hodgkins lymphoma, bladder melanoma, renal carcinoma, breast carcinoma, prostate carcinoma, ovarian carcinoma or colorectal carcinoma. In accordance with the invention, the pharmaceutical compositions can be used in the treatment of a wide variety of cancers, including but not limited to solid tumors (e.g., tumors of the head and neck, lung, breast, colon, prostate, bladder, rectum, brain, gastric tissue, bone, ovary, thyroid, or endometrium), hematological malignancies (e.g., leukemias, lymphomas, myelomas), carcinomas (e.g. bladder carcinoma, renal carcinoma, breast carcinoma, colorectal carcinoma), neuroblastoma, or melanoma. Non-limiting examples of these cancers include diffuse large B-cell lymphoma (DLBCL), T-cell lymphomas or leukemias, e.g., cutaneous T-cell lymphoma (CTCL), noncutaneous peripheral T-cell lymphoma, lymphoma associated with human T-cell lymphotrophic virus (HTLV), adult T-cell leukemia/lymphoma (ATLL), as well as acute lymphocytic leukemia, acute nonlymphocytic leukemia, acute myeloid leukemia, chronic lymphocytic leukemia, chronic myelogenous leukemia, Hodgkin's disease, non-Hodgkin's lymphoma, myeloma, multiple myeloma, mesothelioma, childhood solid tumors, brain neuroblastoma, retinoblastoma, glioma, Wilms' tumor, bone cancer and soft-tissue sarcomas, common solid tumors of adults such as head and neck cancers (e.g., oral, laryngeal and esophageal), genitourinary cancers (e.g., prostate, bladder, renal, uterine, ovarian, testicular, rectal, and colon), lung cancer (e.g., small cell carcinoma and non-small cell lung carcinoma, including squamous cell carcinoma and adenocarcinoma), breast cancer, pancreatic cancer, melanoma and other skin cancers, basal cell carcinoma, metastatic skin carcinoma, squamous cell carcinoma of both ulcerating and papillary type, stomach cancer, brain cancer, liver cancer, adrenal cancer, kidney cancer, thyroid cancer, medullary carcinoma, osteosarcoma, soft-tissue sarcoma, Ewing's sarcoma, veticulum cell sarcoma, and Kaposi's sarcoma. Also included are pediatric forms of any of the cancers described herein.

## Methods of Administration

In all of the methods described herein, the pharmaceutical composition may be administered orally in a gelatin capsule. The composition may be administered in unit dosages according to the methods described herein once-daily, twice-daily or three times-daily.

The daily administration is then repeated continuously for a period of several days to several years. Oral treatment may continue for between one week and the life of the patient. In one embodiment, the administration takes place for five consecutive days after which time the patient can be evaluated to determine if further administration is required. The administration can be continuous or intermittent, i.e., treatment for a number of consecutive days followed

by a rest period.

The pharmaceutical compositions of the present invention may be administered at orally at a total daily dose of between 25 to 4000 mg/$m^2$, for example, about 25 to 1000 mg, 50-1000 mg, 100 mg, 200 mg, 300 mg, 400 mg, 600 mg, 800 mg, 1000 mg and the like. Typically the compound is administered as a single dose when administering up to 400 mg to the patient. For higher total dosages (i.e., greater than 400 mg), the total is split into multiple dosages, for example, twice daily, three times daily or the like, or spread out over equal periods of time during the day. For example, two doses, e.g., 500 mg each, can be administered 12 hours apart to achieve a total dosage of 1000 mg in a day.

In one embodiment, SAHA is administered to the patient at a total daily dosage of 200 mg. In another embodiment, SAHA is administered to the patient at a total daily dosage of 400 mg. In another embodiment, SAHA is administered to the patient at a total daily dosage of 600 mg.

In one embodiment, the amount of the HDAC inhibitor administered to the patient is less than an amount that would cause unmanageable toxicity in the patient. In certain embodiments, the amount of the HDAC inhibitor that is administered to the patient is less than the amount that causes a concentration of the compound in the patient's plasma to equal or exceed the toxic level of the compound. In one embodiment, the concentration of the HDAC inhibitor in the patient's plasma is maintained at between about 10 nM to about 5000 nM. The optimal amount of the HDAC inhibitor that should be administered to the patient in the practice of the present invention will depend on the particular compound used and the type of cancer being treated.

Histone Deacetylases and Histone Deacetylase Inhibitors

Histone deacetylases (HDACs), as that term is used herein, are enzymes that catalyze the removal of acetyl groups from lysine residues in the amino terminal tails of the nucleosomal core histones. As such, HDACs together with histone acetyl transferases (HATs) regulate the acetylation status of histones. Histone acetylation affects gene expression and inhibitors of HDACs, such as the hydroxamic acid-based hybrid polar compound suberoylanilide hydroxamic acid (SAHA) induce growth arrest, differentiation and/or apoptosis of transformed cells *in vitro* and inhibit tumor growth *in vivo.* HDACs can be divided into three classes based on structural homology. Class I HDACs (HDACs 1, 2, 3 and 8) bear similarity to the yeast RPD3 protein, are located in the nucleus and are found in complexes associated with transcriptional co-repressors. Class II HDACs (HDACs 4, 5, 6, 7 and 9) are similar to the yeast HDA1 protein, and have both nuclear and cytoplasmic subcellular localization. Both Class I and II HDACs are inhibited by hydroxamic acid-based HDAC inhibitors, such as SAHA. Class III HDACs form a structurally distant class of NAD dependent enzymes that are related to the yeast SIR2 proteins and are not inhibited by hydroxamic acid-based HDAC inhibitors.

Histone deacetylase inhibitors or HDAC inhibitors, as that term is used herein are compounds that are capable of inhibiting the deacetylation of histones *in vivo, in vitro* or both. As such, HDAC inhibitors inhibit the activity of at least one histone deacetylase. As a result of inhibiting the deacetylation of at least one histone, an increase in acetylated histone occurs and accumulation of acetylated histone is a suitable biological marker for assessing the activity of HDAC inhibitors. Therefore, procedures that can assay for the accumulation of acetylated histones can be used to determine the HDAC inhibitory activity of compounds of interest. It is understood that compounds that can inhibit histone deacetylase activity can also bind to other substrates and as such can inhibit other biologically active molecules such as enzymes. It is also to be understood that the compounds of the present invention are capable of inhibiting any of the histone deacetylases set forth above, or any other histone deacetylases.

For example, in patients receiving HDAC inhibitors, the accumulation of acetylated histones in peripheral mononuclear cells as well as in tissue treated with HDAC inhibitors can be determined against a suitable control.

HDAC inhibitory activity of a particular compound can be determined *in vitro* using, for example, an enzymatic assay which shows inhibition of at least one histone deacetylase. Further, determination of the accumulation of acetylated histones in cells treated with a particular composition can be determinative of the HDAC inhibitory activity of a compound.

Assays for the accumulation of acetylated histones are well known in the literature. See, for example, Marks, P.A. et al., J. Natl. Cancer Inst., 92:1210-1215, 2000, Butler, L.M. et al., Cancer Res. 60:5165-5170 (2000), Richon, V. M. et al., Proc. Natl. Acad. Sci., USA, 95:3003-3007, 1998, and Yoshida, M. et al., J. Biol. Chem., 265:17174-17179, 1990.

For example, an enzymatic assay to determine the activity of a histone deacetylase inhibitor compound can be conducted as follows. Briefly, the effect of an HDAC inhibitor compound on affinity purified human epitope-tagged (Flag) HDAC1 can be assayed by incubating the enzyme preparation in the absence of substrate on ice for about 20 minutes with the indicated amount of inhibitor compound. Substrate ([$^3$H]acetyl-labelled murine erythroleukemia cell-derived histone) can be added and the sample can be incubated for 20 minutes at 37°C in a total volume of 30 μL. The reaction can then be stopped and released acetate can be extracted and the amount of radioactivity release determined by scintillation counting. An alternative assay useful for determining the activity of a histone deacetylase inhibitor compound is the "HDAC Fluorescent Activity Assay; Drug Discovery Kit-AK-500" available from BIOMOL® Research Laboratories, Inc., Plymouth Meeting, PA.

In vivo studies can be conducted as follows. Animals, for example, mice, can be injected intraperitoneally with

an HDAC inhibitor compound. Selected tissues, for example, brain, spleen, liver etc, can be isolated at predetermined times, post administration. Histones can be isolated from tissues essentially as described by Yoshida et al., J. Biol. Chem. 265:17174-17179, 1990. Equal amounts of histones (about 1 $\mu$g) can be electrophoresed on 15% SDS-polyacrylamide gels and can be transferred to Hybond-P filters (available from Amersham). Filters can be blocked with 3% milk and can be probed with a rabbit purified polyclonal anti-acetylated histone H4 antibody ($\alpha$Ac-H4) and anti-acetylated histone H3 antibody ($\alpha$Ac-H3) (Upstate Biotechnology, Inc.). Levels of acetylated histone can be visualized using a horseradish peroxidase-conjugated goat anti-rabbit antibody (1:5000) and the SuperSignal chemiluminescent substrate (Pierce). As a loading control for the histone protein, parallel gels can be run and stained with Coomassie Blue (CB).

In addition, hydroxamic acid-based HDAC inhibitors have been shown to up regulate the expression of the $p21^{WAF1}$ gene. The $p21^{WAF1}$ protein is induced within 2 hours of culture with HDAC inhibitors in a variety of transformed cells using standard methods. The induction of the $p21^{WAF1}$ gene is associated with accumulation of acetylated histones in the chromatin region of this gene. Induction of $p21^{WAF1}$ can therefore be recognized as involved in the G1 cell cycle arrest caused by HDAC inhibitors in transformed cells.

Typically, HDAC inhibitors fall into five general classes: 1) hydroxamic acid derivatives; 2) Short-Chain Fatty Acids (SCFAs); 3) cyclic tetrapeptides; 4) benzamides; and 5) electrophilic ketones.

Thus, the present invention includes within its broad scope compositions comprising HDAC inhibitors which are 1) hydroxamic acid derivatives; 2) Short-Chain Fatty Acids (SCFAs); 3) cyclic tetrapeptides; 4) benzamides; 5) electrophilic ketones; and/or any other class of compounds capable of inhibiting histone deacetylases, for use in inhibiting histone deacetylase, inducing terminal differentiation in neoplastic cells, and /or inducing differentiation of tumor cells in a tumor.

Examples of such HDAC inhibitors include, but are not limited to:

A. Hydroxamic Acid Derivatives such as suberoylanilide hydroxamic acid (SAHA) (Richon et al., Proc. Natl. Acad. Sci. USA 95,3003-3007 (1998)); m-carboxycinnamic acid bishydroxamide (CBHA) (Richon *et al.,* supra); pyroxamide; trichostatin analogues such as trichostatin A (TSA) and trichostatin C (Koghe et al. 1998. Biochem. Pharmacol. 56: 1359-1364); salicylihydroxamic acid (SBHA) (Andrews et al., International J. Parasitology 30,761-768 (2000)); suberoyl bishydroxamic acid (SBHA) (U.S. Patent No. 5,608,108); azelaic bishydroxamic acid (ABHA) (Andrews *et al.,* supra); azelaic-1-hydroxamate-9-anilide (AAHA) (Qiu et al., Mol. Biol. Cell 11, 2069-2083 (2000)); 6-(3-chlorophenylureido) carpoic hydroxamic acid (3C1-UCHA); oxamflatin [(2E)-5-[3-[(phenylsufonyl) aminol phenyl]-pent-2-en-4-ynohydroxamic acid] (Kim et al. Oncogene, 18: 2461 2470 (1999)); A-161906, Scriptaid (Su et al. 2000 Cancer Research, 60: 3137-3142); PXD-101 (Prolifix); LAQ-824; CHAP; MW2796 (Andrews *et al.,* supra); MW2996 (Andrews *et al.,* supra); or any of the hydroxamic acids disclosed in U.S. Patent Numbers 5,369,108, 5,932,616, 5,700,811, 6,087,367 and 6,511, 990.

B. Cyclic Tetrapeptides such as trapoxin A (TPX)-cyclic tetrapeptide (cyclo-(L- phenylalanyl-L-phenylalanyl-D-pipecolinyl-L-2-amino-8-oxo-9,10-epoxy decanoyl)) (Kijima et al., J Biol. Chem. 268,22429-22435 (1993)); FR901228 (FK 228, depsipeptide) (Nakajima et al., Ex. Cell Res. 241,126-133 (1998)); FR225497 cyclic tetrapeptide (H. Mori et al., PCT Application WO 00/08048 (17 February 2000)); apicidin cyclic tetrapeptide [cyclo(NO-methyl-L-tryptophanyl-L -isoleucinyl-D-pipecolinyl-L-2-amino-8-oxodecanoyl)] (Darkin-Rattray et al., Proc. Natl. Acad. Sci. USA 93,1314313147 (1996)); apicidin Ia, apicidin Ib, apicidin Ic, apicidin IIa, and apicidin IIb (P. Dulski et al., PCT Application WO 97/11366); CHAP, HC-toxin cyclic tetrapeptide (Bosch et al., Plant Cell 7, 1941-1950 (1995)); WF27082 cyclic tetrapeptide (PCT Application WO 98/48825); and chlamydocin (Bosch *et al.,* supra).

C. Short chain fatty acid (SCFA) derivatives such as: sodium butyrate (Cousens et al., J. Biol. Chem. 254, 1716-1723 (1979)); isovalerate (McBain et al., Biochem. Pharm. 53: 1357-1368 (1997)); valerate (McBain *et al.,* supra) ; 4-phenylbutyrate (4-PBA) (Lea and Tulsyan, Anticancer Research, 15,879-873 (1995)); phenylbutyrate (PB) (Wang et al., Cancer Research, 59, 2766-2799 (1999)); propionate (McBain *et al.,* supra); butyramide (Lea and Tulsyan, supra); isobutyramide (Lea and Tulsyan, supra); phenylacetate (Lea and Tulsyan, supra); 3-bromopropionate (Lea and Tulsyan, supra); tributyrin (Guan et al., Cancer Research, 60,749-755 (2000)); valproic acid and valproate.

D. Benzamide derivatives such as CI-994; MS-27-275 [N- (2-aminophenyl)-4- [N- (pyridin-3-yl methoxycarbonyl) aminomethyl] benzamide] (Saito et al., Proc. Natl. Acad. Sci. USA 96, 4592-4597 (1999)); and 3'-amino derivative of MS-27-275 (Saito *et al.,* supra).

E. Electrophilic ketone derivatives such as trifluoromethyl ketones (Frey et al, Bioorganic & Med. Chem. Lett. (2002), 12, 3443-3447; U.S. 6,511,990) and $\alpha$-keto amides such as N-methyl- $\alpha$-ketoamides

F. Other HDAC Inhibitors such as depudecin (Kwon et al. 1998. PNAS 95: 3356-3361.

In one embodiment, hydroxamic acid based HDAC inhibitors are suberoylanilide hydroxamic acid (SAHA), m-carboxycinnamic acid bishydroxamate (CBHA) and pyroxamide. SAHA has been shown to bind directly in the catalytic pocket of the histone deacetylase enzyme. SAHA induces cell cycle arrest, differentiation and/or apoptosis of transformed cells in culture and inhibits tumor growth in rodents. SAHA is effective at inducing these effects in both solid tumors and

hematological cancers. It has been shown that SAHA is effective at inhibiting tumor growth in animals with no toxicity to the animal. The SAHA-induced inhibition of tumor growth is associated with an accumulation of acetylated histones in the tumor. SAHA is effective at inhibiting the development and continued growth of carcinogen-induced (N-methylnitrosourea) mammary tumors in rats. SAHA was administered to the rats in their diet over the 130 days of the study. Thus, SAHA is a nontoxic, orally active antitumor agent whose mechanism of action involves the inhibition of histone deacetylase activity. In other embodiments, HDAC inhibitors are those disclosed in U.S. Patent Numbers 5,369,108, 5,932,616, 5,700,811, 6,087,367 and 6,511, 990.

Combination Therapy

The methods of the present invention may also comprise initially administering to the subject an antitumor agent so as to render the neoplastic cells in the subject resistant to an antitumor agent and subsequently administering an effective amount of any of the compositions of the present invention, effective to selectively induce terminal differentiation, cell growth arrest and/or apoptosis of such cells.

The antitumor agent may be one of numerous chemotherapy agents such as an alkylating agent, an antimetabolite, a hormonal agent, an antibiotic, colchicine, a vinca alkaloid, L-asparaginase, procarbazine, hydroxyurea, mitotane, nitrosoureas or an imidazole carboxamide. Suitable agents are those agents that promote depolarization of tubulin. In one embodiment, the antitumor agent is colchicine or a vinca alkaloid; vinblastine or vincristine. In embodiments where the antitumor agent is vincristine, the cells preferably are treated so that they are resistant to vincristine at a concentration of about 5 mg/ml. The treating of the cells to render them resistant to an antitumor agent may be effected by contacting the cells with the agent for a period of at least 3 to 5 days. The contacting of the resulting cells with any of the compounds above is performed as described previously. In addition to the above chemotherapy agents, the compounds may also be administered together with radiation therapy.

*Alkylating Agents*

Alkylating agents react with nucleophilic residues, such as the chemical entities on the nucleotide precursors for DNA production. They affect the process of cell division by alkylating these nucleotides and preventing their assembly into DNA.

Examples of alkylating agents include, but are not limited to, bischloroethylamines (nitrogen mustards, e.g., chlorambucil, cyclophosphamide, ifosfamide, mechlorethamine, melphalan, uracil mustard), aziridines (e.g., thiotepa), alkyl alkone sulfonates (e.g., busulfan), nitrosoureas (e.g., carmustine, lomustine, streptozocin), nonclassic alkylating agents (altretamine, dacarbazine, and procarbazine), platinum compounds (carboplastin and cisplatin). These compounds react with phosphate, amino, hydroxyl, sulfihydryl, carboxyl, and imidazole groups.

Under physiological conditions, these drugs ionize and produce positively charged ion that attach to susceptible nucleic acids and proteins, leading to cell cycle arrest and/or cell death. The alkylating agents are cell cycle phase nonspecific agents because they exert their activity independently of the specific phase of the cell cycle. The nitrogen mustards and alkyl alkone sulfonates are most effective against cells in the G1 or M phase. Nitrosoureas, nitrogen mustards, and aziridines impair progression from the G1 and S phases to the M phases. Chabner and Collins eds. (1990) "Cancer Chemotherapy: Principles and Practice", Philadelphia: JB Lippincott.

The alkylating agents are active against wide variety of neoplastic diseases, with significant activity in the treatment of leukemias and lymphomas as well as solid tumors. Clinically this group of drugs is routinely used in the treatment of acute and chronic leukemias; Hodgkin's disease; non-Hodgkin's lymphoma; multiple myeloma; primary brain tumors; carcinomas of the breast, ovaries, testes, lungs, bladder, cervix, head and neck, and malignant melanoma.

The major toxicity common to all of the alkylating agents is myelosuppression. Additionally, Gastrointestinal adverse effects of variable severity occur commonly and various organ toxicities are associated with specific compounds. Black and Livingston (1990) Drugs 39: 489-501 and 39: 652-673.

*Antibiotics*

Antibiotics (e.g., cytotoxic antibiotics) act by directly inhibiting DNA or RNA synthesis and are effective throughout the cell cycle. Examples of antibiotic agents include anthracyclines (e.g., doxorubicin, daunorubicin, epirubicin, idarubicin and anthracenedione), mitomycin C, bleomycin, dactinomycin, plicatomycin. These antibiotic agents interfere with cell growth by targeting different cellular components. For example, anthracyclines are generally believed to interfere with the action of DNA topoisomerase II in the regions of transcriptionally active DNA, which leads to DNA strand scissions.

Bleomycin is generally believed to chelate iron and forms an activated complex, which then binds to bases of DNA, causing strand scissions and cell death.

The antibiotic agents have been used as therapeutics across a range of neoplastic diseases, including carcinomas of the breast, lung, stomach and thyroids, lymphomas, myelogenous leukemias, myelomas, and sarcomas. The primary toxicity of the anthracyclines within this group is myelosuppression, especially granulocytopenia. Mucositis often accompanies the granulocytopenia and the severity correlates with the degree of myelosuppression. There is also significant cardiac toxicity associated with high dosage administration of the anthracyclines.

*Antimetabolic Agents*

Antimetabolic agents (i.e., antimetabolites) are a group of drugs that interfere with metabolic processes vital to the physiology and proliferation of cancer cells. Actively proliferating cancer cells require continuous synthesis of large quantities of nucleic acids, proteins, lipids, and other vital cellular constituents.

Many of the antimetabolites inhibit the synthesis of purine or pyrimidine nucleosides or inhibit the enzymes of DNA replication. Some antimetabolites also interfere with the synthesis of ribonucleosides and RNA and/or amino acid metabolism and protein synthesis as well. By interfering with the synthesis of vital cellular constituents, antimetabolites can delay or arrest the growth of cancer cells. Examples of antimetabolic agents include, but are not limited to, fluorouracil (5-FU), floxuridine (5-FUdR), methotrexate, leucovorin, hydroxyurea, thioguanine (6-TG), mercaptopurine (6-MP), cytarabine, pentostatin, fludarabine phosphate, cladribine (2-CDA), asparaginase, and gemcitabine.

Antimetabolic agents have been widely used to treat several common forms of cancer including carcinomas of colon, rectum, breast, liver, stomach and pancreas, malignant melanoma, acute and chronic leukemia and hair cell leukemia. Many of the adverse effects of antimetabolite treatment result from suppression of cellular proliferation in mitotically active tissues, such as the bone marrow or gastrointestinal mucosa. Patients treated with these agents commonly experience bone marrow suppression, stomatitis, diarrhea, and hair loss. Chen and Grem (1992) Curr. Opin. Oncol. 4: 1089-1098.

*Hormonal Agents*

The hormonal agents are a group of drug that regulate the growth and development of their target organs. Most of the hormonal agents are sex steroids and their derivatives and analogs thereof, such as estrogens, progestogens, anti-estrogens, androgens, antiandrogens and progestins. These hormonal agents may serve as antagonists of receptors for the sex steroids to down regulate receptor expression and transcription of vital genes. Examples of such hormonal agents are synthetic estrogens (e.g., diethylstibestrol), antiestrogens (e.g., tamoxifen, toremifene, fluoxymesterol and raloxifene), antiandrogens (bicalutamide, nilutamide, flutamide), aromatase inhibitors (e.g., aminoglutethimide, anastrozole and tetrazole), luteinizing hormone release hormone (LHRH) analogues, ketoconazole, goserelin acetate, leuprolide, megestrol acetate and mifepristone.

Hormonal agents are used to treat breast cancer, prostate cancer, melanoma and meningioma. Because the major action of hormones is mediated through steroid receptors, 60% receptor-positive breast cancer responded to first-line hormonal therapy; and less than 10% of receptor-negative tumors responded. The main side effect associated with hormonal agents is flare. The frequent manifestations are an abrupt increase of bony pain, erythema around skin lesions, and induced hypercalcemia.

Specifically, progestogens are used to treat endometrial cancers, since these cancers occur in women that are exposed to high levels of oestrogen unopposed by progestogen.

Antiandrogens are used primarily for the treatment of prostate cancer, which is hormone dependent. They are used to decrease levels of testosterone, and thereby inhibit growth of the tumor.

Hormonal treatment of breast cancer involves reducing the level of oestrogen-dependent activation of oestrogen receptors in neoplastic breast cells. Anti-oestrogens act by binding to oestrogen receptors and prevent the recruitment of coactivators, thus inhibiting the oestrogen signal.

LHRH analogues are used in the treatment of prostate cancer to decrease levels of testosterone and so decrease the growth of the tumor.

Aromatase inhibitors act by inhibiting the enzyme required for hormone synthesis. In post-menopausal women, the main source of oestrogen is through the conversion of androstenedione by aromatase.

*Plant-derived Agents*

Plant-derived agents are a group of drugs that are derived from plants or modified based on the molecular structure of the agents. They inhibit cell replication by preventing the assembly of the cell's components that are essential to cell division.

Examples of plant derived agents include vinca alkaloids (e.g., vincristine, vinblastine, vindesine, vinzolidine and vinorelbine), podophyllotoxins (e.g., etoposide (VP-16) and teniposide (VM-26)), taxanes (e.g., paclitaxel and docetaxel). These plant-derived agents generally act as antimitotic agents that bind to tubulin and inhibit mitosis. Podophyllotoxins such as etoposide are believed to interfere with DNA synthesis by interacting with topoisomerase II, leading to DNA strand scission.

Plant-derived agents are used to treat many forms of cancer. For example, vincristine is used in the treatment of the leukemias, Hodgkin's and non-Hodgkin's lymphoma, and the childhood tumors neuroblastoma, rhabdomyosarcoma, and Wilms' tumor. Vinblastine is used against the lymphomas, testicular cancer, renal cell carcinoma, mycosis fungoides, and Kaposi's sarcoma. Doxetaxel has shown promising activity against advanced breast cancer, non-small cell lung cancer (NSCLC), and ovarian cancer.

Etoposide is active against a wide range of neoplasms, of which small cell lung cancer, testicular cancer, and NSCLC are most responsive.

The plant-derived agents cause significant side effects on patients being treated. The vinca alkaloids display different spectrum of clinical toxicity. Side effects of vinca alkaloids include neurotoxicity, altered platelet function, myelosuppression, and leukopenia. Paclitaxel causes dose-limiting neutropenia with relative sparing of the other hematopoietic cell lines. The major toxicity of the epipophyllotoxins is hematologic (neutropenia and thrombocytopenia).

Other side effects include transient hepatic enzyme abnormalities, alopecia, allergic reactions, and peripheral neuropathy.

*Biologic Agents*

Biologic agents are a group of biomolecules that elicit cancer/tumor regression when used alone or in combination with chemotherapy and/or radiotherapy. Examples of biologic agents include immuno-modulating proteins such as cytokines, monoclonal antibodies against tumor antigens, tumor suppressor genes, and cancer vaccines.

Cytokines possess profound immunomodulatory activity. Some cytokines such as interleukin-2 (IL-2, aldesleukin) and interferon-$\alpha$ (IFN-$\alpha$) demonstrated antitumor activity and have been approved for the treatment of patients with metastatic renal cell carcinoma and metastatic malignant melanoma. IL-2 is a T-cell growth factor that is central to T-cell-mediated immune responses. The selective antitumor effects of IL-2 on some patients are believed to be the result of a cell-mediated immune response that discriminate between self and nonself.

Interferon-$\alpha$ includes more than 23 related subtypes with overlapping activities. IFN-$\alpha$ has demonstrated activity against many solid and hematologic malignancies, the latter appearing to be particularly sensitive.

Examples of interferons include, interferon-$\alpha$, interferon-$\beta$ (fibroblast interferon) and interferon-$\gamma$ (fibroblast interferon). Examples of other cytokines include erythropoietin (epoietin-$\alpha$), granulocyte-CSF (filgrastin), and granulocyte, macrophage-CSF (sargramostim). Other immuno-modulating agents other than cytokines include bacillus Calmette-Guerin, levamisole, and octreotide, a long-acting octapeptide that mimics the effects of the naturally occuring hormone somatostatin.

Furthermore, the anti-cancer treatment can comprise treatment by immunotherapy with antibodies and reagents used in tumor vaccination approaches. The primary drugs in this therapy class are antibodies, alone or carrying e.g. toxins or chemostherapeutics/cytotoxics to cancer cells. Monoclonal antibodies against tumor antigens are antibodies elicited against antigens expressed by tumors, preferably tumor-specific antigens. For example, monoclonal antibody HERCEPTIN® (trastuzumab) is raised against human epidermal growth factor receptor2 (HER2) that is overexpressed in some breast tumors including metastatic breast cancer. Overexpression of HER2 protein is associated with more aggressive disease and poorer prognosis in the clinic. HERCEPTIN® is used as a single agent for the treatment of patients with metastatic breast cancer whose tumors overexpress the HER2 protein.

Another example of monoclonal antibodies against tumor antigens is RITUXAN® (rituximab) that is raised against CD20 on lymphoma cells and selectively deplete normal and malignant CD20+ pre-B and mature B cells.

RITUXAN is used as single agent for the treatment of patients with relapsed or refractory low-grade or follicular, CD20+, B cell non-Hodgkin's lymphoma. MYELOTARG® (gemtuzumab ozogamicin) and CAMPATH® (alemtuzumab) are further examples of monoclonal antibodies against tumor antigens that may be used.

Tumor suppressor genes are genes that function to inhibit the cell growth and division cycles, thus preventing the development of neoplasia. Mutations in tumor suppressor genes cause the cell to ignore one or more of the components of the network of inhibitory signals, overcoming the cell cycle checkpoints and resulting in a higher rate of controlled cell growth-cancer. Examples of the tumor suppressor genes include Duc-4, NF-1, NF-2, RB, p53, WT1, BRCA1 and BRCA2.

DPC4 is involved in pancreatic cancer and participates in a cytoplasmic pathway that inhibits cell division. NF-1 codes for a protein that inhibits Ras, a cytoplasmic inhibitory protein. NF-1 is involved in neurofibroma and pheochromocytomas of the nervous system and myeloid leukemia. NF-2 encodes a nuclear protein that is involved in meningioma, schwanoma, and ependymoma of the nervous system. RB codes for the pRB protein, a nuclear protein that is a major inhibitor of cell cycle. RB is involved in retinoblastoma as well as bone, bladder, small cell lung and breast cancer. P53 codes for p53 protein that regulates cell division and can induce apoptosis. Mutation and/or inaction of p53 is found in a wide ranges of cancers. WTI is involved in Wilms' tumor of the kidneys. BRCA1 is involved in breast and ovarian

cancer, and BRCA2 is involved in breast cancer. The tumor suppressor gene can be transferred into the tumor cells where it exerts its tumor suppressing functions.

Cancer vaccines are a group of agents that induce the body's specific immune response to tumors. Most of cancer vaccines under research and development and clinical trials are tumor-associated antigens (TAAs). TAAs are structures (i.e., proteins, enzymes or carbohydrates) that are present on tumor cells and relatively absent or diminished on normal cells. By virtue of being fairly unique to the tumor cell, TAAs provide targets for the immune system to recognize and cause their destruction. Examples of TAAs include gangliosides (GM2), prostate specific antigen (PSA), α-fetoprotein (AFP), carcinoembryonic antigen (CEA) (produced by colon cancers and other adenocarcinomas, e.g., breast, lung, gastric, and pancreatic cancers), melanoma-associated antigens (MART-1, gap100, MAGE 1,3 tyrosinase), papillomavirus E6 and E7 fragments, whole cells or portions/lysates of autologous tumor cells and allogeneic tumor cells.

*Other Therapies*

Recent developments have introduced, in addition to the traditional cytotoxic and hormonal therapies used to treat cancer, additional therapies for the treatment of cancer. For example, many forms of gene therapy are undergoing preclinical or clinical trials.

In addition, approaches are currently under development that is based on the inhibition of tumor vascularization (angiogenesis). The aim of this concept is to cut off the tumor from nutrition and oxygen supply provided by a newly built tumor vascular system.

In addition, cancer therapy is also being attempted by the induction of terminal differentiation of the neoplastic cells. Suitable differentiation agents include the compounds disclosed in any one or more of the following references.

a) Polar compounds (Marks et al (1987); , Friend, C., Scher, W., Holland, J. W., and Sato, T. (1971) Proc. Natl. Acad. Sci. (USA) 68: 378-382; Tanaka, M., Levy, J., Terada, M., Breslow, R., Rifkind, R. A., and Marks, P. A. (1975) Proc. Natl. Acad. Sci. (USA) 72: 1003-1006; Reuben, R. C., Wife, R. L., Breslow, R., Rifkind, R. A., and Marks, P. A. (1976) Proc. Natl. Acad. Sci. (USA) 73: 862-866);

b) Derivatives of vitamin D and retinoic acid (Abe, E., Miyaura, C., Sakagami, H., Takeda, M., Konno, K., Yamazaki, T., Yoshika, S., and Suda, T. (1981) Proc. Natl. Acad. Sci. (USA) 78: 4990-4994; Schwartz, E. L., Snoddy, J. R., Kreutter, D., Rasmussen, H., and Sartorelli, A. C. (1983) Proc. Am. Assoc. Cancer Res. 24: 18; Tanenaga, K., Hozumi, M., and Sakagami, Y. (1980) Cancer Res. 40: 914-919);

c) Steroid hormones (Lotem, J. and Sachs, L. (1975) Int. J. Cancer 15: 731-740);

d) Growth factors (Sachs, L. (1978) Nature (Lond.) 274: 535, Metcalf, D. (1985) Science, 229: 16-22);

e) Proteases (Scher, W., Scher, B. M., and Waxman, S. (1983) Exp. Hematol. 11: 490-498; Scher, W., Scher, B. M., and Waxman, S. (1982) Biochem. & Biophys. Res. Comm. 109: 348-354);

f) Tumor promoters (Huberman, E. and Callaham, M. F. (1979) Proc. Natl. Acad. Sci. (USA) 76: 1293-1297; Lottem, J. and Sachs, L. (1979) Proc. Natl. Acad. Sci. (USA) 76: 5158-5162); and

g) inhibitors of DNA or RNA synthesis (Schwartz, E. L. and Sartorelli, A. C. (1982) Cancer Res. 42: 2651-2655, Terada, M., Epner, E., Nudel, U., Salmon, J., Fibach, E., Rifkind, R. A., and Marks, P. A. (1978) Proc. Natl. Acad. Sci. (USA) 75: 2795-2799; Morin, M. J. and Sartorelli, A. C. (1984) Cancer Res. 44: 2807-2812; Schwartz, E. L., Brown, B. J., Nierenberg, M., Marsh, J. C., and Sartorelli, A. C. (1983) Cancer Res. 43: 2725-2730; Sugano, H., Furusawa, M., Kawaguchi, T., and Ikawa, Y. (1973) Bibl. Hematol. 39: 943-954; Ebert, P. S., Wars, I., and Buell, D. N. (1976) Cancer Res. 36: 1809-1813; Hayashi, M., Okabe, J., and Hozumi, M. (1979) Gann 70: 235-238),

The combination of the pharmaceutical compositions of this invention and any of the anti-cancer agents described above and their use thereof are within the scope of the present invention.

The invention is illustrated in the examples in the Experimental Details Section which follows. This section is set forth to aid in an understanding of the invention but is not intended to, and should not be construed to limit in any way the invention as set forth in the claims which follow thereafter.

EXPERIMENTAL DETAILS SECTION

EXAMPLE 1

Production of SAHA Form I

Step 1 8-anilino-8-oxooctanoic acid, suberanilic acid (Compound 3)

Suberic acid (Compound 1, 174.2 g, 1.0 mole), aniline (Compound 2, 85.8-94.9 g), and toluene (0.1-0.2 L) are

combined, heated to reflux and refluxed for a minimum of 60 hours. The reaction is quenched at reflux by adjusting the pH to ≥ 11 with 10% sodium hydroxide solution. The aqueous phase is separated. The organic layer is combined with toluene (0.11-0.13 L) and water (0.3-0.4 L), and the aqueous layer is separated. The aqueous layers from the extractions and toluene (0.11-0.13 L) are combined, settled, and then separated. The aqueous layer is extracted twice with toluene (0.2-0.3 L) at 60-70°C. The aqueous layer is adjusted at 20-30°C to a pH of 5.8-6.2, using hydrochloric acid and 10% sodium hydroxide solution as needed. The batch is filtered, washed with chilled water (0.2-0.3 L) and then washed with chilled isopropanol. The wet cake is dried at a maximum of 65°C under vacuum to yield suberanilic acid.

Step 2 methyl 8-anilino-8-oxooctanoate; methyl suberanilate (Compound 4)

Suberanilic acid (Compound 3, 249.3 g, 1.0 mole) and methanol (0.4-0.5 L) are combined and heated to 45-55°C. The pH is adjusted to ≤ 2 using hydrochloric acid, and the batch temperature is maintained at 45-55°C until the reaction is complete. The reaction is quenched with deionized water (0.1-0.2 L). The batch is cooled to 25-30°C and seeded to induce crystallization, and then cooled to 0-10°C. The batch is filtered, and the cake washed with a 50:50 (v/v) methanol/water solution (0.28-0.34 L) at 0-10°C. The wet cake is dried at a maximum of 46°C under vacuum to yield methyl suberanilate.

Step 3 *N*-hydroxy-*N'*-phenyloctanediamide; vorinostat (Compound 5)

Methyl suberanilate (Compound 4, 263.3 g, 1.0 mole) and 2M hydroxylamine freebase solution (0.8-1.0 L) are combined. While maintaining the batch at a maximum of 20°C, the apparent pH is adjusted to ≥ 10.5 with sodium methoxide in methanol as needed. While maintaining the batch at maximum 20°C and apparent pH ≥ 10.5 using sodium methoxide in methanol, the batch is aged. During the age, hydroxylamine freebase solution (0.5-0.6 L) is added, and the batch is maintained at maximum 20°C and apparent pH ≥ 10.5 until the reaction is complete. The reaction is quenched by adding the batch to water (0.9-1.1 L) while maintaining the batch temperature between 20-35°C, and the water content of the batch is adjusted to 35-45%. The pH is adjusted to 8.8-9.2 using glacial acetic acid and sodium carbonate as needed. The batch is cooled to 0-10°C over 5-10 hours. The batch is filtered and the cake washed with 55:45 (v/v) methanol/water (0.45-0.6 L) at 0-10°C. The wet cake is vacuum conditioned until the water content is ≤ 35%.

The vorinostat crude (264.32 g, 1.0 mole) wet cake is combined with denatured ethanol (1308-1599 g) and water (167-204 g). Hydroxylamine hydrochloride (> 9 mEquiv) and sodium methoxide in methanol (> 9 mEquiv) are added to the slurry, and the batch is heated to 70-80°C. The solution is filtered and then crystallized by slowly cooling to 0-10°C. The batch is filtered and the cake washed with cold 4:1 (v/v) denatured ethanol/water. The wet cake is dried at a maximum of 45°C under vacuum.

Step 4 N-hydroxy-N'-phenyloctanediamide - vorinostat-fine (wet-milled) (Compound 6)

Vorinostat (Compound 5, 264.3 g, 1.0 mole) is slurried in a 50:50 (v/v) ethanol/water solution (minimum 2.8 L). The vorinostat slurry is wet-milled to a mean size of 25-45 μm while maintaining the batch temperature at 7-30°C. The final slurry is filtered and the wet cake is washed with 0-40°C water (minimum 0.8 L). The wet cake is dried at a maximum of 55°C under vacuum to a maximum water content of 0.2% (w/w) to yield vorinostat-fine drug substance.

Step 5 N-hydroxy-N'-phenyloctanediamide - vorinostat-coarse (Compound 7)

Vorinostat (Compound 5, 264.3 g, 1.0 mole) is slurried in a 50:50 (v/v) ethanol/water solution (4.9-5.5 L). Under a minimum of 15 psig pressure, the slurry is heated to 65-70°C to dissolve and then cooled to 60-64°C. A seed slurry is transferred into the batch while maintaining the batch temperature. The batch is aged for a minimum of 2 hours at 61-63°C. The batch is cooled in three steps by controlling the jacket temperature: (1) to 55°C at 0.35-0.78°C/hour, (2) to 45°C at 0.83-2.00°C/hour, and (3) to -5 to 25°C at 2.00-4.44°C/hour. The final slurry is aged at -5 to 25°C for about 1 hour and then filtered. The wet cake is washed with water (minimum 0.8 L). The wet cake is dried at a maximum of 55°C under vacuum to yield vorinostat-coarse drug substance.

The seed slurry is prepared by combining vorinostat-fine dry cake (97.8-116.3 g, 0.37-0.44 mol) and 50:50 (v/v) ethanol/water solution (1.0-1.2 L). Under a minimum of 15 psig pressure, the seed slurry is heated to 62-66°C, aged for about 0.5 hours and then cooled to 60-64°C.

**EXAMPLE 2**

Powder Blending of SAHA Crystals

Powder Blending

30% of the Vorinostat-fine crystals are blended with 70% of the Vorinostat-coarse crystals. 25.0 Kg of blended SAHA Polymorph I crystals were first sieved through a 30 mesh screen (600 μm). The resulting SAHA, 11.1 Kg of Microcrystalline Cellulose (Avicel PH-101), and 1.13 Kg of Croscarmellose Sodium were then loaded into the 141.6 L V-blender, 113 L Tote blender or another comparable sized and type blender. For the V-blender, the resulting material was mixed to homogeneity for approximately 8 minutes at approximately 25 rpm. For the Tote blender, the resulting material was mixed to homogeneity for approximately 17 minutes at approximately 12 rpm.

Powder Blend Lubrication

293.0 g of Magnesium Stearate (vegetable grade) was sieved through a 30 mesh screen (600 μm) and loaded into the V-blender with the blended powder mixture. The resulting mixture was blended to homogeneity for approximately 8 minutes at approximately 25 rpm. 293.0 g of Magnesium Stearate (vegetable grade) was also sieved through a 60 mesh screen (250 μm) and loaded into a tote blender with the blended powder mixture. The resulting mixture was blended to homogeneity for approximately 17 minutes at approximately 12 rpm.

Table 1 summarizes the physical properties of the raw materials in the capsule.

Table 1: Physical and Chemical Properties of Raw Materials.

| Raw Material | Physical Property | Value |
|---|---|---|
| Suberoylanilide hydroxamic acid (SAHA) - fine and coarse crystals | Melting Point (DSC)<br>Solubility:<br>o In Water<br>o In Methanol<br>o In Ethanol<br>o 2% CIP 100 aqueous soln.<br>o 2% SD-20 aqueous soln. | 161 - 163°C<br><br>< 0.1 mg/ml<br>42 mg/ml<br>0.1 mg/ml<br>11.3 mg/ml<br>0.085 mg/ml |
| Microcrystalline Cellulose (Avicel PH-101) NF, Ph. Eur., JP (FMC BioPolymer) | Nominal Mean Particle Size<br>Moisture Content<br>Bulk Density | 50 μm<br>≤ 5%<br>0.26 - 0.31 g/cc |
| Croscarmellose Sodium NF, Ph. Eur., JP (FMC BioPolymer) | Bulk Density<br>Tapped Density<br>Particle Size Distribution | 0.48 g/cc<br>0.67 g/cc<br>≤ 2% wt. retained on Mesh No. 200 (75 μm)<br>≤ 10% wt. retained on Mesh No. 325 (45 μm) |
| Magnesium Stearate (vegetable grade) NF, Ph. Eur., JP (Mallinckrodt Baker Inc.) | Bulk Density<br>Particle Size Distribution<br><br>Specific Surface Area | 0.16 g/cc<br>≤ 2% wt. retained on Mesh No. 200 (75 μm)<br>$4.2 \pm 0.04$ $m^2/g$ |

EXAMPLE 3

Encapsulation of SAHA Capsules

Encapsulation/Weight Sorting

The lubed powder mixture was encapsulated using an H&K encapsulator, polished tamping pins or chromium nitride coated tamping pins and size "3" capsules to the desired capsule weight. The filled capsules were polished using a capsule polisher and subsequently weight sorted using a weight sorter to the appropriate weight limit range. Table 2 summarizes the encapsulator settings.

Table 2. Summary of Encapsulator Operational Settings

| | |
|---|---|
| Dosing Disc | 10.0-12.7 mm |
| Tamping Pins/Station | 3 or 12 |
| Tamping Pin Type | Polished Uncoated or chromium nitride coated |
| Vacuum | ON |
| Encapsulator Speed | 150-270 caps/min or 750-1000 caps/min |

The final SAHA Capsule Composition is illustrated in Table 3. The capsules are weight-sorted using an acceptance limit for capsule weight variation of ± 10% the target capsule weight. The capsule weight variation in a typical batch is ± 4% of the target capsule weight.

Table 3: SAHA Capsule Composition

| Ingredient | Unit Weight (mg) | Weight (%) |
|---|---|---|
| Suberoylanilide hydroxamic acid (SAHA) - Fine | X | Y |
| Suberoylanilide hydroxamic acid (SAHA) - Coarse | 100.0 - x | 66.67 - y |
| Microcrystalline Cellulose (Avicel PH-101) NF, Ph. Eur., JP | 44.33 | 29.80 |
| Croscarmellose | 4.500 | 3.00 |
| Sodium NF, Ph. Eur., JP | | |
| Magnesium Stearate (vegetable grade) NF, Ph. Eur., JP | 1.170 | 0.78 |
| Hard Gelatin Capsule, Size "3" Conisnap, White Opaque/White Opaque* | 49.00 | N/A |
| Total** | 150.0 | 100.00 |
| *The market capsule ink formulation is Colorcon S-1-17762. TSE-free gelatin capsules.<br>** Total weights do not include the hard gelatin capsule shells. | | |

**EXAMPLE 4**

Measurement of Dissolution Rate of Capsules containg SAHA

The dissolution rate of SAHA from hard gelatin capsules is evaluated using a USP Dissolution Apparatus II (VK 7000, Varian Inc., Cary, NC). Each capsule is placed into a helical sinker (Quality Lab Accessories L.L.C., Manville, NJ) and delivered to vessels containing 900 mL of 2.0% Tween (TCI America, Portland, Oregon) at a temperature of 37±0.5°C. The paddles are rotated at 100 rpm and samples were pulled at specified time intervals via an autosampler (VK 8000, Varian Inc., Cary, NC) equipped with 35 μm full flow filters (Varian Inc., Cary, NC).

Subsequently, samples were assayed for SAHA by High Performance Liquid Chromatography (Agilent 1100 series, Agilent Technologies Inc., Wilmington, DE). The chromatographic analysis was conducted using a Phenomenex Luna C8 (2) (100 x 4.6 mm) 5 μm particle size column, a mobile phase of 1:1 methanol/0.1% trifluroacetic acid (Reagent Grade, Fisher), and a detection wavelength of 242 nm.

**EXAMPLE 5**

X-Ray Powder Diffraction Analysis of SAHA

X-ray Powder Diffraction analysis was performed on SAHA Form I obtained in accordance with Example 1, and on SAHA Form II-V prepared by methods detailed in Table 4 below.

Table 4: SAHA Samples analyzed by X-ray Powder Diffraction

| SAHA Sample | Reference | Method |
|---|---|---|
| SAHA Form I | - | Example 1 |
| SAHA Form II | U.S. 5,369,108 Columns 25-26 Procedures A, C, D | SAHA was dissolved in EtOAc/THF (3/1). The solutions were passed through a plug of silica gel using EtOAc/THF (3/1). Fractions were collected and concentrated. The solid appeared pink. |
| SAHA Form III | U.S. 5,369,108 Columns 25-26 Procedure B | SAHA was dissolved in methanol, filtered via celite, and concentrated on the rotovap to dryness. The residues were slurried with hexanes and filtered. The solids appeared pink. |
| SAHA Form IV | Mai et al OPPI Briefs (2001)Vol 33(4), 391-394 | SAHA was recrystallized from acetonitrile. |
| SAHA Form V | Stowell et al J. Med. Chem. (1995), 38(8), 1411-1413 | To a mixture of SAHA (4.0g) in anhydrous methanol (15 mL) was added NaOMe (10.7 mL, 4.37 M, 47 mmol). The solution became homogeneous, but solid formed after about 5 minutes. The mixture was stirred for 15 min, and then 100 ml of water was added followed by slow addition of glacial acetic acid (3.77 mL, 4.0 g). The crystalline solid was collected and washed with water (2x75 mL). The solid was dried under high vaccum overnght yielding 3.85 g (96% recovery) of an off-white solid. |

X-Ray Diffraction Analysis:

The samples were analyzed on a Siemens D500 Automated Powder Diffractometer (Instrument ID No. LD-301-4), which is operated according to Standard Operating Procedure EQ-27, Rev. 12, in accordance with the manufacturer's instructions. The Diffractometer is equipped with a graphite monochromator and a Cu ($\lambda$=1.54A) X-ray source operated at 50kV, 40 mA. Two-theta calibration is performed using an NBS mica standard (SRM675). The samples were analyzed using the following instrument parameters:

| | |
|---|---|
| Measuring Range: | 4-40 2 theta |
| Step Width: | 0.05 Å |
| Measuring Time per Step: | 1.2 seconds |

Sample preparation was performed according to Standard Operating Procedure MIC-7, Rev. 2 (Section 3.1.2), in accordance with the manufacturer's instructions, using a zero background sample plate (#1). The samples were processed following a light mortar and pestle grind to ensure homogeneity.

Figure 3A-E depicts the X-ray diffractograms for SAHA Forms I-V. The corresponding data for the X-ray diffractograms is presented in Tables 5-9 below:

Table 5: SAHA Form I

| Peak | 2Theta (deg) | D (Å) |
|---|---|---|
| 1 | 8.97 | 9.86159 |
| 2 | 9.37 | 9.43 |
| 3 | 17.46 | 5.07 |
| 4 | 19.41 | 4.57 |
| 5 | 20.04 | 4.43 |
| 6 | 23.96 | 3.71 |
| 7 | 24.44 | 3.64 |
| 8 | 24.76 | 3.59 |
| 9 | 24.96 | 3.56 |
| 10 | 27.96 | 3.19 |
| 11 | 43.29 | 2.08 |

Table 6: SAHA Form II

| Peak | 2Theta (deg) | D (Å) | Peak | 2Theta (deg) | D (Å) |
|---|---|---|---|---|---|
| 1 | 5.12 | 17.24 | 18 | 21.72 | 4.09 |
| 2 | 5.46 | 16.15 | 19 | 22.07 | 4.02 |
| 3 | 7.48 | 11.8 | 20 | 22.88 | 3.88 |
| 4 | 7.72 | 11.44 | 21 | 23.36 | 3.80 |
| 5 | 8.15 | 18.84 | 22 | 23.79 | 3.73 |
| 6 | 8.72 | 10.13 | 23 | 24.16 | 3.68 |
| 7 | 9.21 | 9.59 | 24 | 24.66 | 3.61 |
| 8 | 10.91 | 8.09 | 25 | 25.75 | 3.46 |
| 9 | 12.38 | 7.14 | 26 | 26.92 | 3.31 |
| 10 | 13.55 | 6.52 | 27 | 27.56 | 3.23 |
| 11 | 17.31 | 5.12 | 28 | 27.88 | 3.20 |
| 12 | 18.22 | 4.86 | 29 | 28.53 | 3.12 |
| 13 | 18.86 | 4.70 | 30 | 30.68 | 2.91 |
| 14 | 19.32 | 4.59 | 31 | 40.21 | 2.24 |
| 15 | 19.88 | 4.46 | 32 | 42.80 | 2.11 |
| 16 | 20.76 | 4.27 | 33 | 43.16 | 2.09 |
| 17 | 21.20 | 4.19 | | | |

Table 7: SAHA Form III

| Peak | 2Theta (deg) | D (Å) | Peak | 2Theta (deg) | D (Å) |
|---|---|---|---|---|---|
| 1 | 10.10 | 8.75 | 12 | 23.81 | 3.73 |
| 2 | 12.13 | 7.29 | 13 | 24.54 | 3.62 |

(continued)

| Peak | 2Theta (deg) | D (Å) | Peak | 2Theta (deg) | D (Å) |
|---|---|---|---|---|---|
| 3 | 13.83 | 6.40 | 14 | 25.04 | 3.55 |
| 4 | 15.11 | 5.86 | 15 | 25.36 | 3.51 |
| 5 | 17.65 | 5.02 | 16 | 26.10 | 3.41 |
| 6 | 18.54 | 4.78 | 17 | 26.80 | 3.32 |
| 7 | 18.80 | 4.71 | 18 | 35.62 | 2.51 |
| 8 | 19.60 | 4.52 | 19 | 37.12 | 2.42 |
| 9 | 20.18 | 4.40 | 20 | 40.92 | 2.20 |
| 10 | 20.90 | 4.25 | 21 | 42.43 | 2.13 |
| 11 | 21.69 | 4.10 | 22 | 44.83 | 2.02 |

Table 8: SAHA Form IV

| Peak | 2Theta (deg) | D (Å) |
|---|---|---|
| 1 | 8.84 | 9.99 |
| 2 | 9.25 | 9.55 |
| 3 | 11.00 | 8.04 |
| 4 | 12.44 | 7.11 |
| 5 | 17.38 | 5.10 |
| 6 | 19.37 | 4.58 |
| 7 | 19.93 | 4.45 |
| 8 | 22.36 | 3.97 |
| 9 | 22.89 | 3.88 |
| 10 | 23.83 | 3.73 |
| 11 | 24.24 | 3.67 |
| 12 | 24.80 | 3.59 |
| 13 | 25.80 | 3.45 |
| 14 | 26.96 | 3.30 |
| 15 | 27.84 | 3.20 |
| 16 | 28.39 | 3.14 |

Table 9: SAHA Form V

| Peak | 2Theta (deg) | D (Å) |
|---|---|---|
| 1 | 5.08 | 17.39 |
| 2 | 9.20 | 9.60 |
| 3 | 10.07 | 8.77 |
| 4 | 12.13 | 7.29 |
| 5 | 15.09 | 5.86 |
| 6 | 17.65 | 5.02 |

(continued)

| Peak | 2Theta (deg) | D (Å) |
|---|---|---|
| 7 | 19.32 | 4.59 |
| 8 | 19.80 | 4.48 |
| 9 | 20.16 | 4.41 |
| 10 | 20.87 | 4.25 |
| 11 | 21.67 | 4.10 |
| 12 | 24.56 | 3.62 |
| 13 | 25.25 | 3.52 |
| 14 | 26.10 | 3.41 |
| 15 | 35.62 | 2.51 |
| 16 | 37.12 | 2.42 |
| 17 | 40.90 | 2.20 |
| 18 | 41.78 | 2.16 |
| 19 | 42.42 | 2.13 |
| 20 | 44.82 | 2.02 |

The X-ray powder diffraction pattern of SAHA Form I was also collected using a X'PERT Pro Phillips X-ray diffractometer with a copper Kα radiation (wavelength 1.542 Å). The prominent 2θ positions along with the *d*-spacings are summarized in Table 5A.

Table 5A SAHA Form I

| Position [°2θ] | d-spacing [Å] |
|---|---|
| 9.1 | 9.7 |
| 10.8 | 8.2 |
| 12.3 | 7.2 |
| 17.2 | 5.2 |
| 19.2 | 4.6 |
| 19.8 | 4.5 |
| 23.7 | 3.7 |
| 24.1 | 3.7 |
| 25.7 | 3.5 |
| 26.8 | 3.3 |
| 27.7 | 3.2 |

EXAMPLE 6

Preparation of Iron SAHA Metal Chelate Complex

Chemical Reaction Scheme

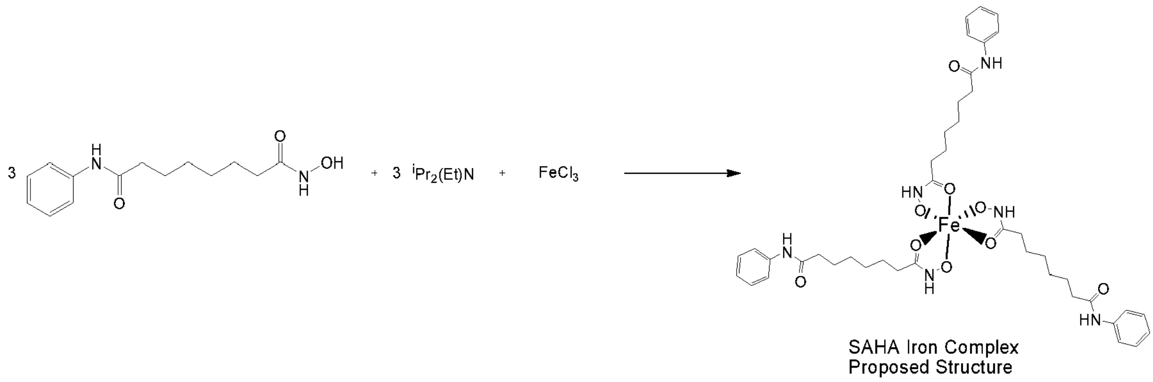


Experimental

At ambient temperature, SAHA (1.0 g, 3.78 mmol) and ethanol (10.0 mL) were added to an erlenmyer flask, and N,N-diisopropylethylamine was then added dropwise (0.66 mL, 3.78 mmol) with stirring. To the resultant slurry, a solution of ferric chloride in ethanol (0.205 g, 1.26 mmol in 10.0 mL of ethanol) was added. The mixture immediately took on a deep red color as the ferric chloride solution was added. Ethanol (5.0 mL) was used to wash the remaining ferric chloride solution into the reaction flask. After 5 min of stirring at ambient temperature, the reaction mixture became a clear, dark red solution. The reaction mixture was allowed to stir at ambient temperature for 16 h. After this time, a dark orange precipitate was observed. The orange solid was collected via filtration and washed with ethanol (3 x 3.0 mL). The filtrate was colorless. The orange solid was allowed to dry for 24 h (mass 1.02 g, 96 % yield).

Analysis

Elemental analysis for C, H, N and Fe content is provided below in Table 10:

Table 10: Elemental Analysis

| % Element | Theoretical | Actual |
|---|---|---|
| % C | 59.64 | 58.29 |
| %H | 6.79 | 6.88 |
| %N | 9.94 | 9.56 |
| %Fe | 6.60 | 6.3 |

The results of the elemental analysis are in good agreement with the proposed structure shown above.

EXAMPLE 7

X-Ray Powder Diffraction Analysis of iron SAHA chelate complex

The X-ray powder diffraction pattern (Figure 2) of the crystalline iron SAHA chelate complex was generated on a Philips Analytical X'Pert PRO X-ray Diffraction System with PW3040/60 console. A PW3373/00 ceramic Cu LEF X-ray tube K-Alpha radiation was used as the source. The crystalline iron SAHA chelate complex can be characeterized at 8.8, 14.5 and 21.8 degrees (2θ). The crystalline iron SAHA chelate complex is further characterized by peaks at 13.3, 20.3 and 24.6 degrees (2θ). Additional peaks attributed to the complex are observed at 18.5, 25.8 and 33.3 degrees (2θ).

EXAMPLE 8

Patient Studies

Patients with cancer are administered the pharmaceutical composition comprising a metal HDAC inhibitor chelate complex, or a pharmaceutical composition of an HDAC inhibitor and chelatable metal compound. Adverse experiences of the patients are recorded and compared with adverse experiences of patients administered with the

HDAC inhibitor.

Patients with either Cutaneous T-cell Lymphoma or Peripheral T-cell Lymphoma are administered the pharmaceutical composition once daily continuously, wherein the amount of SAHA administered is 400 mg. Patients with either Cutaneous T-cell Lymphoma or Peripheral T-cell Lymphoma are administered the pharmaceutical composition twice daily three to five days per week, wherein the amount of SAHA administered for each dose is 300 mg. Patients with advanced leukemias and myelodysplatic syndrome (MDS) are administered the pharmaceutical composition orally (po) three times (tid) a day for 14 days followed by 1 week of rest, for a 3-week course, wherein the amount of SAHA administered for each dose is at 100 mg, 150 mg, 200 mg or 250 mg.

Patients with cancer are co-administered an HDAC inhibitor and chelatable metal compound. Adverse experiences of the patients are recorded and compared with adverse experiences of patients administered with only the HDAC inhibitor.

Patients with either Cutaneous T-cell Lymphoma or Peripheral T-cell Lymphoma are co-administered orally SAHA once daily continuously at 400 mg or at 300 mg twice daily three to five days per week and an iron or zinc supplement daily. Patients with advanced leukemias and myelodysplatic syndrome (MDS) are administered orally SAHA (po) at 100 mg, 150 mg, 200 mg or 250 mg three times (tid) a day for 14 days followed by 1 week of rest and an iron or zinc supplement daily, for a 3-week course. Patients with advanced cancer including hematologic cancer and solid tumors are administered orally SAHA 400 or 500 mg once a day daily, 200 or 300 mg twice a day daily, 300 mg or 400 mg twice a day intermittently 3 days a week, or 100 mg three times a day (2 wks) and an iron or zinc supplement daily. Patients with Diffuse Large B Cell Lymphoma (DLBCL) or mesothelioma are administered orally SAHA 300 mg twice a day 3 days a week and an iron or zinc supplement daily. The amount of zinc or iron administered will be expected to vary from patient to patient and will be the amount needed to alleviate the HDAC inhibitor induced toxicities without causing metal overload mediated toxicity.

## EXAMPLE 9

### Effects of Zinc Supplementation on Vorinostat-Mediated Side Effects

To test whether the maximum tolerated dose (MTD) of vorinostat in mice can be increased with zinc supplementation, Mice (CD1 nu/nu female, 6-8 weeks old, Charles River Laboratories) are administered vehicle, 150 mg/kg (MTD) or 300 mg/kg vorinostat in vehicle (Table 11). Mice are given 0.5, 1, or 5 molar equivalents (relative to vorinostat) of zinc chloride. This corresponds to $ZnCl_2$ doses of 38.5, 77, and 385 mg/kg for the 150 mg/kg vorinostat group.

An initial study of non-tumored mice is used to establish MTD of vorinostat/Zn combinations. If a shift in MTD is noted, a subsequent experiment with mice is used to establish HCT116 colon carcinoma subcutaneous (s.q.) xenografts to evaluate the role of altered MTD on tumor growth (efficacy).

Table 11. Vorinostat MTD and Zinc Supplementation. All mice are dosed (i.p.) daily (qd) for 14 days with the indicated amounts of compounds.

| Group | N | Agent | Mg / kg | Agent | Mg / kg |
|---|---|---|---|---|---|
| 1 | 5 | Vehicle | - | - | - |
| 2 | 5 | Vehicle | - | $ZnCl_2$ | 38.5 |
| 3 | 5 | Vehicle | - | $ZnCl_2$ | 77 |
| 4 | 5 | Vehicle | - | $ZnCl_2$ | 385 |
| 5 | 5 | Vorinostat | 150 | - | - |
| 6 | 5 | Vorinostat | 150 | $ZnCl_2$ | 38.5 |
| 7 | 5 | Vorinostat | 150 | $ZnCl_2$ | 77 |
| 8 | 5 | Vorinostat | 150 | $ZnCl_2$ | 385 |
| 9 | 5 | Vorinostat | 300 | - | - |
| 10 | 5 | Vorinostat | 300 | $ZnCl_2$ | 77 |
| 11 | 5 | Vorinostat | 300 | $ZnCl_2$ | 154 |
| 12 | 5 | Vorinostat | 300 | $ZnCl_2$ | 770 |

The vehicle for Vorinostat (Dose IP at 10 ul/g) contain 10% v/v DMSO (Sigma, cat # : D8418), 45% v/v PEG-400 and 45% water. Vorinostat in vehicle (Dose IP at 10 ul/g) is prepared by weighing the compound (pre-warm each vehicle component to 37°C prior to formulating the compound), adding 1/10$^{th}$ final volume of 100% DMSO, sonicating until clear solution, adding 50% PEG-400 (in water) to final desired volume, sonicating and maintaining at 37°C at all times. $ZnCl_2$ is dissolved in sterile phosphate buffered saline (PBS).

Body weight and survival are monitored as indications of toxicity. Doses resulting in a group average body weight loss of more than 20% average will be considered non-tolerated. Likewise, doses resulting in the treatment-related death of more than 2 mice per group will be considered non-tolerated. For tumored animals, tumor sizes in response to treatment are measured by callipering 2-3 times per week.

Table 12. End of Study Serum Harvest Schedule

| First 2 mice in each group | Last 3 mice in each group |
|---|---|
| RO 0.5 hr PD, CP 4 hr PD | RO 2 hr PD, CP 24 hr |
| PD = post-dose; RO= retro-orbital bleed; CP= cardiac puncture (terminal) | |

Retro-orbital bleed: 0.2 mL blood is collected by retro-orbital bleed under isoflurane anesthesia. Blood is processed for serum (no anti-coagulant added).

Cardiac Puncture: Full volume of blood is collected by terminal cardiac puncture under $CO_2$ anesthesia. Blood is processed for serum (no anti-coagulant added).

Complete Blood Count (CBC)

Vorinostat treatment has been associated with reduced numbers of certain blood populations, including platelets and lymphocytes. CBC from mice are monitored before (baseline) and during (Day7), and after (Day14) treatment. A small quantity (<50 μl) of blood is obtained by RO bleed subsets counted by an Advia 120 instrument (Seimens Medical).

Autopsy and tissue harvest

For tumored animals, the xenograft tumor is removed, weighed, and portions flash frozen or fixed in buffered formalin. Fixed tumor material is processed as paraffin embedded slides and subjected to immunohistochemical analyses. These include assays for proliferation (Ki-67, BrdU), apoptosis (Caspase-3), and acetylation of various histones (H2, H3, H4). Histone protein from frozen material are used to assay histone acetylation changes by ELISA (ezyme-linked immunosorbent assay). Mice are also subjected to a full autopsy.

While this invention has been particularly shown and described with references to embodiments thereof, it will be understood by those skilled in the art that various changes in form and details may be made therein without departing from the meaning of the invention described. Rather, the scope of the invention is defined by the claims that follow.

References

1. Sporn, M. B., Roberts, A. B., and Driscoll, J. S. (1985) in Cancer: Principles and Practice of Oncology, eds. Hellman, S., Rosenberg, S. A., and DeVita, V. T., Jr., Ed. 2, (J. B. Lippincott, Philadelphia), P. 49.

2. Breitman, T. R., Selonick, S. E., and Collins, S. J. (1980) Proc. Natl. Acad. Sci. USA 77: 2936-2940.

3. Olsson, I. L. and Breitman, T. R. (1982) Cancer Res. 42: 3924-3927.

4. Schwartz, E. L. and Sartorelli, A. C. (1982) Cancer Res. 42: 2651-2655.

5. Marks, P. A., Sheffery, M., and Rifkind, R. A. (1987) Cancer Res. 47: 659.

6. Sachs, L. (1978) Nature (Lond.) 274: 535.

7. Friend, C., Scher, W., Holland, J. W., and Sato, T. (1971) Proc. Natl. Acad. Sci. (USA) 68: 378-382.

8. Tanaka, M., Levy, J., Terada, M., Breslow, R., Rifkind, R. A., and Marks, P. A. (1975) Proc. Natl. Acad. Sci. (USA) 72: 1003-1006.

9. Reuben, R. C., Wife, R. L., Breslow, R., Rifkind, R. A., and Marks, P. A. (1976) Proc. Natl. Acad. Sci. (USA) 73: 862-866.

10. Abe, E., Miyaura, C., Sakagami, H., Takeda, M., Konno, K., Yamazaki, T., Yoshika, S., and Suda, T. (1981) Proc. Natl, Acad, Sci. (USA) 78: 4990-4994.

11. Schwartz, E. L., Snoddy, J. R., Kreutter, D., Rasmussen, H., and Sartorelli, A. C. (1983) Proc. Am. Assoc. Cancer Res. 24: 18.

12. Tanenaga, K., Hozumi, M., and Sakagami, Y. (1980) Cancer Res. 40: 914-919.

13. Lotem, J. and Sachs, L. (1975) Int. J. Cancer 15: 731-740.

14. Metcalf, D. (1985) Science, 229: 16-22.

15. Scher, W., Scher, B. M., and Waxman, S. (1983) Exp. Hematol. 11: 490-498.

16. Scher, W., Scher, B. M., and Waxman, S. (1982) Biochem. & Biophys. Res. Comm. 109: 348-354.

17. Huberman, E. and Callaham, M. F. (1979) Proc. Natl. Acad. Sci. (USA) 76: 1293-1297.

18. Lottem, J. and Sachs, L. (1979) Proc. Natl. Acad. Sci. (USA) 76: 5158-5162.

19. Terada, M., Epner, E., Nudel, U., Salmon, J., Fibach, E., Rifkind, R. A., and Marks, P. A. (1978) Proc. Natl. Acad. Sci. (USA) 75: 2795-2799.

20. Morin, M. J. and Sartorelli, A. C. (1984) Cancer Res. 44: 2807-2812.

21. Schwartz, E. L., Brown, B. J., Nierenberg, M., Marsh, J. C., and Sartorelli, A. C. (1983) Cancer Res. 43: 2725-2730.

22. Sugano, H., Furusawa, M., Kawaguchi, T., and Ikawa, Y. (1973) Bibl. Hematol. 39: 943-954.

23. Ebert, P. S., Wars, I., and Buell, D. N. (1976) Cancer Res. 36: 1809-1813.

24. Hayashi, M., Okabe, J., and Hozumi, M. (1979) Gann 70: 235-238.

25. Richon, V.M., Webb, Y., Merger, R., et al. (1996) PNAS 93:5705-8.

26. Cohen, L.A., Amin, S., Marks, P.A., Rifkind, R.A., Desai, D., and Richon, V.M. (1999) Anticancer Research 19: 4999-5006.

27. Grunstein, M. (1997) Nature 389:349-52.

28. Finnin, M.S., Donigian, J.R., Cohen, A., et al. (1999) Nature 401:188-193.

29. Van Lint, C., Emiliani, S., Verdin, E. (1996) Gene Expression 5:245-53.

30. Archer, S. Shufen, M. Shei, A., Hodin, R. (1998) PNAS 95:6791-96.

31. Dressel, U., Renkawitz, R., Baniahmad, A. (2000) Anticancer Research 20(2A):1017-22.

32. Parker, Vigoroux, Reed, AIChE J. (2000) pp. 1290-99.

33. Nunez, Espiell, Chem.Eng.Sci. (1986) pp.2075-83.

34. O. Levenspiel: Chemical Reaction Engineering, 2nd Ed., p. 373.

35. M. Vanni: J. of Colloid and Interface Sci. (2000) pp. 143-160.

36. P. J. Hill and K. M. Ng, AIChE J. (1995) pp. 1204 - 1216.

## Claims

**1.** A pharmaceutical composition comprising suberoylanilide hydroxamic acid (SAHA), or a pharmaceutically acceptable salt or hydrate or solvate thereof, and a chelatable metal compound, wherein the chelatable metal compound comprises zinc or iron.

**2.** A zinc SAHA chelate complex or an iron SAHA chelate complex, or a hydrate or solvate thereof.

**3.** A pharmaceutical composition comprising a zinc SAHA chelate complex or an iron SAHA chelate complex, or a hydrate or solvate thereof of claim 2, and a pharmaceutically acceptable carrier.

**4.** A pharmaceutical composition of claim 1 or 3, for use in treating cancer.

**5.** A pharmaceutical composition of claim 1 or 3, for use in treating cancer and alleviating the side effects of SAHA.

**6.** A zinc SAHA chelate complex or an iron SAHA chelate complex, or a hydrate or solvate thereof of claim 2, for use in treating cancer.

**7.** A zinc SAHA chelate complex or an iron SAHA chelate complex, or a hydrate or solvate thereof of claim 2, for use in treating cancer and alleviating the side effects of SAHA.

**8.** SAHA and a chelatable metal compound for use in treating cancer and alleviating the side effects of SAHA, wherein SAHA and the chelatable metal compound are co-administered, and the chelatable metal compound comprises zinc or iron.

**9.** SAHA and a chelatable metal compound for use in treating cancer and alleviating the side effects of SAHA, wherein the chelatable metal compound comprises zinc or iron, and the chelatable metal compound is administered before or after administration of SAHA.

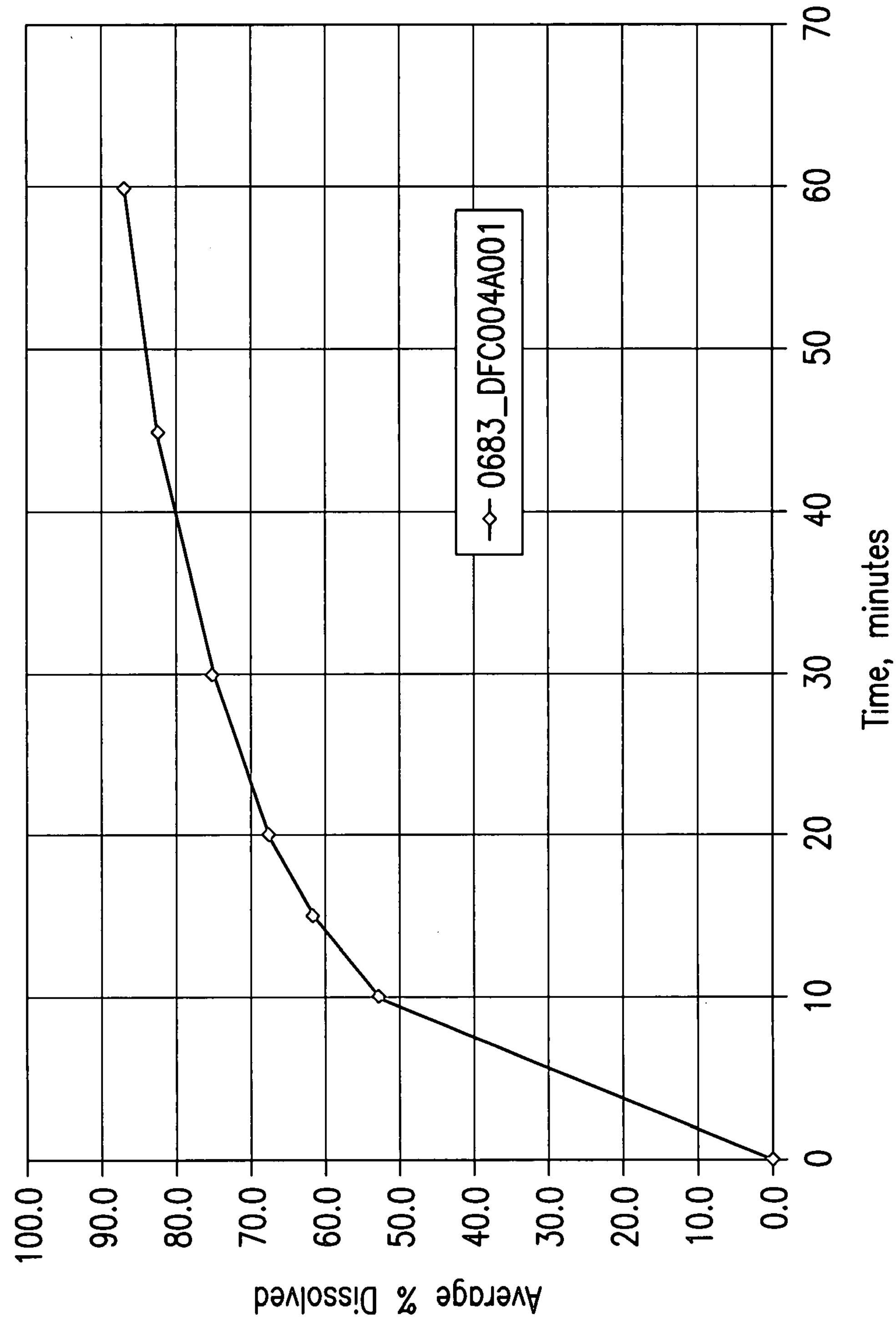
Average % Dissolved
100.0
90.0
80.0
70.0
60.0
50.0
40.0
30.0
20.0
10.0
0.0
0
10
20
30
40
50
60
70
Time, minutes
0683_DFC004A001

FIG. 1

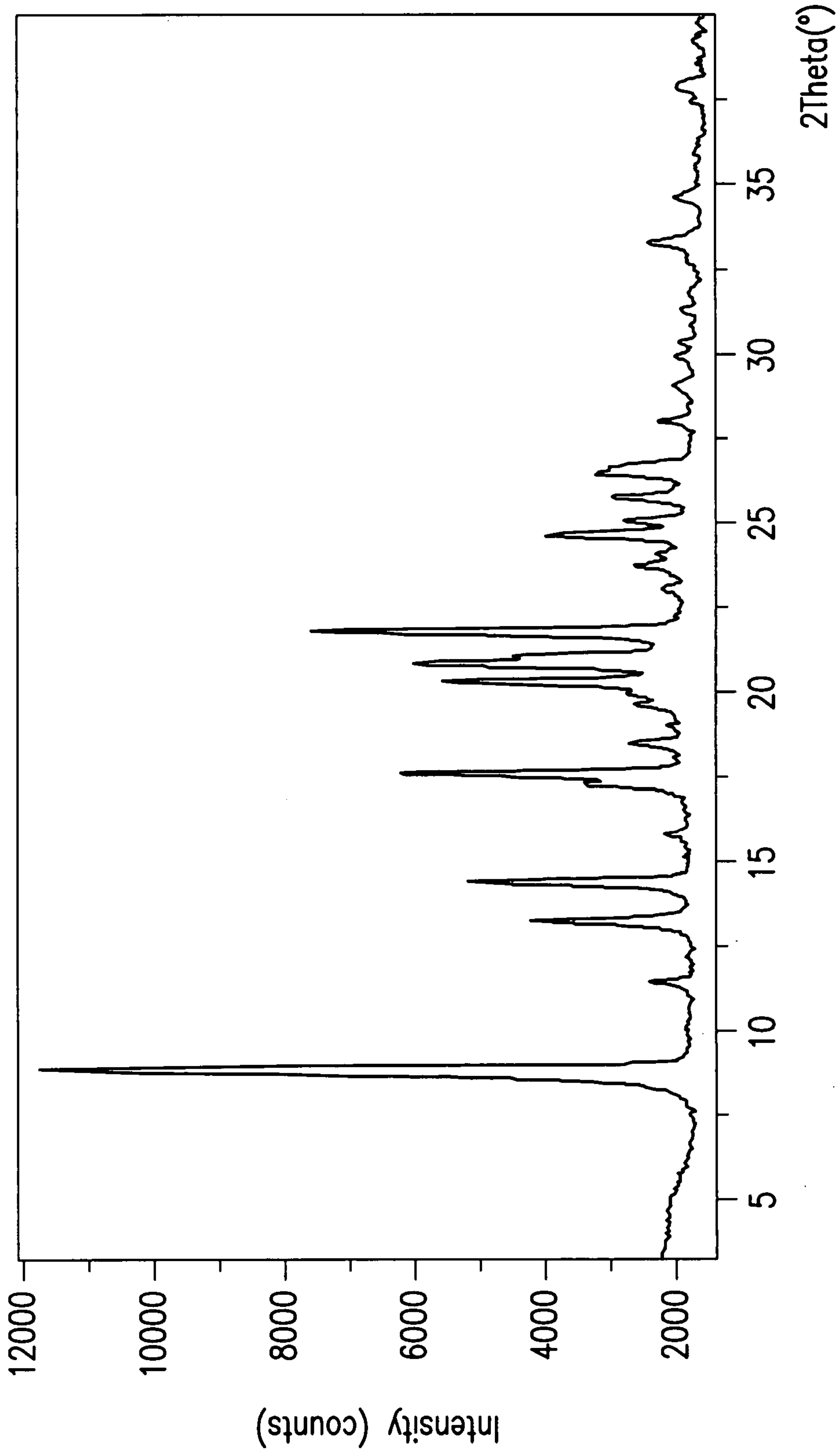
Intensity (counts)
12000
10000
8000
6000
4000
2000
5
10
15
20
25
30
35
2Theta(°)

FIG.2

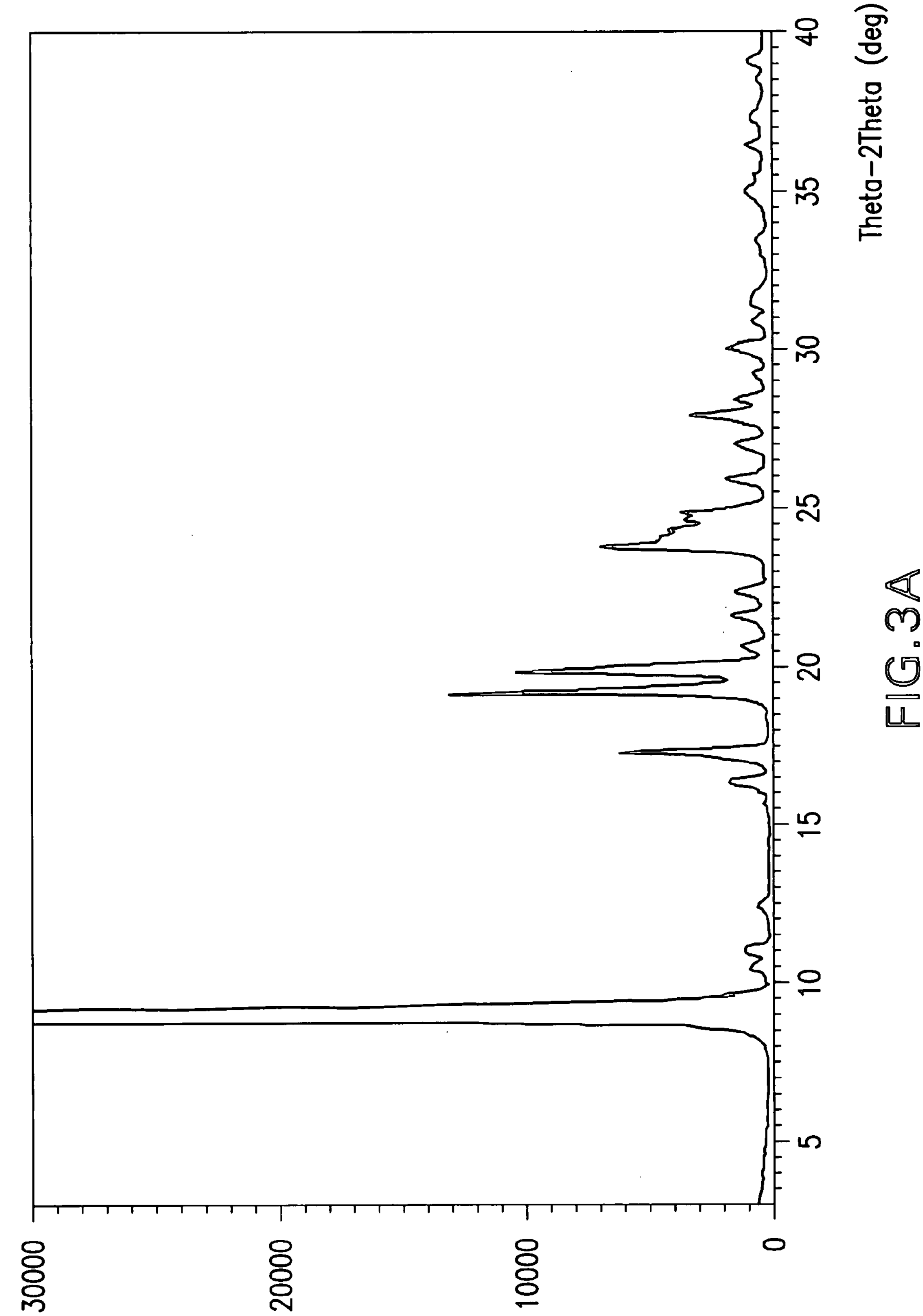


FIG. 3A

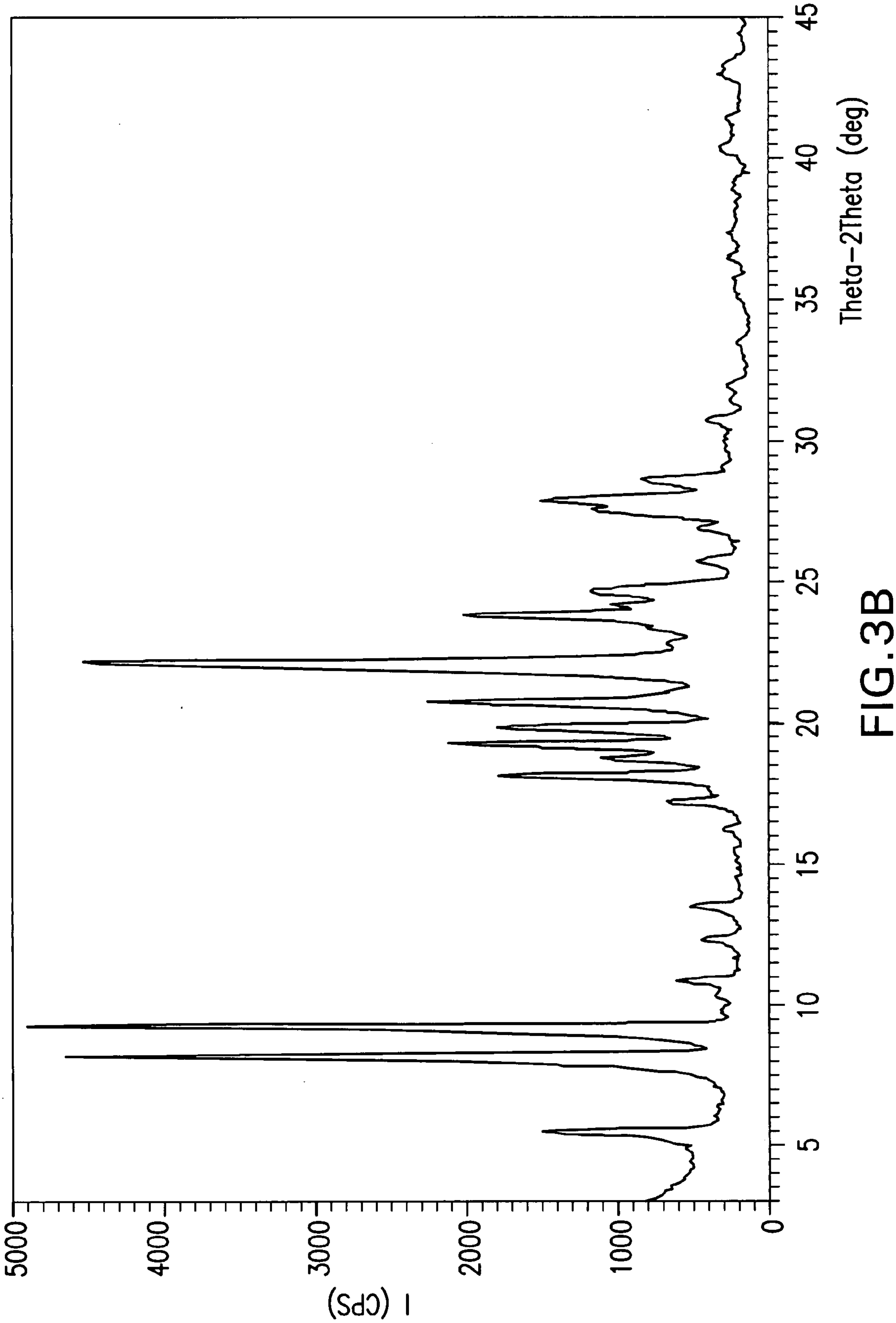
5000
4000
3000
2000
1000
0
I (CPS)
5
10
15
20
25
30
35
40
45
Theta-2Theta (deg)

FIG.3B

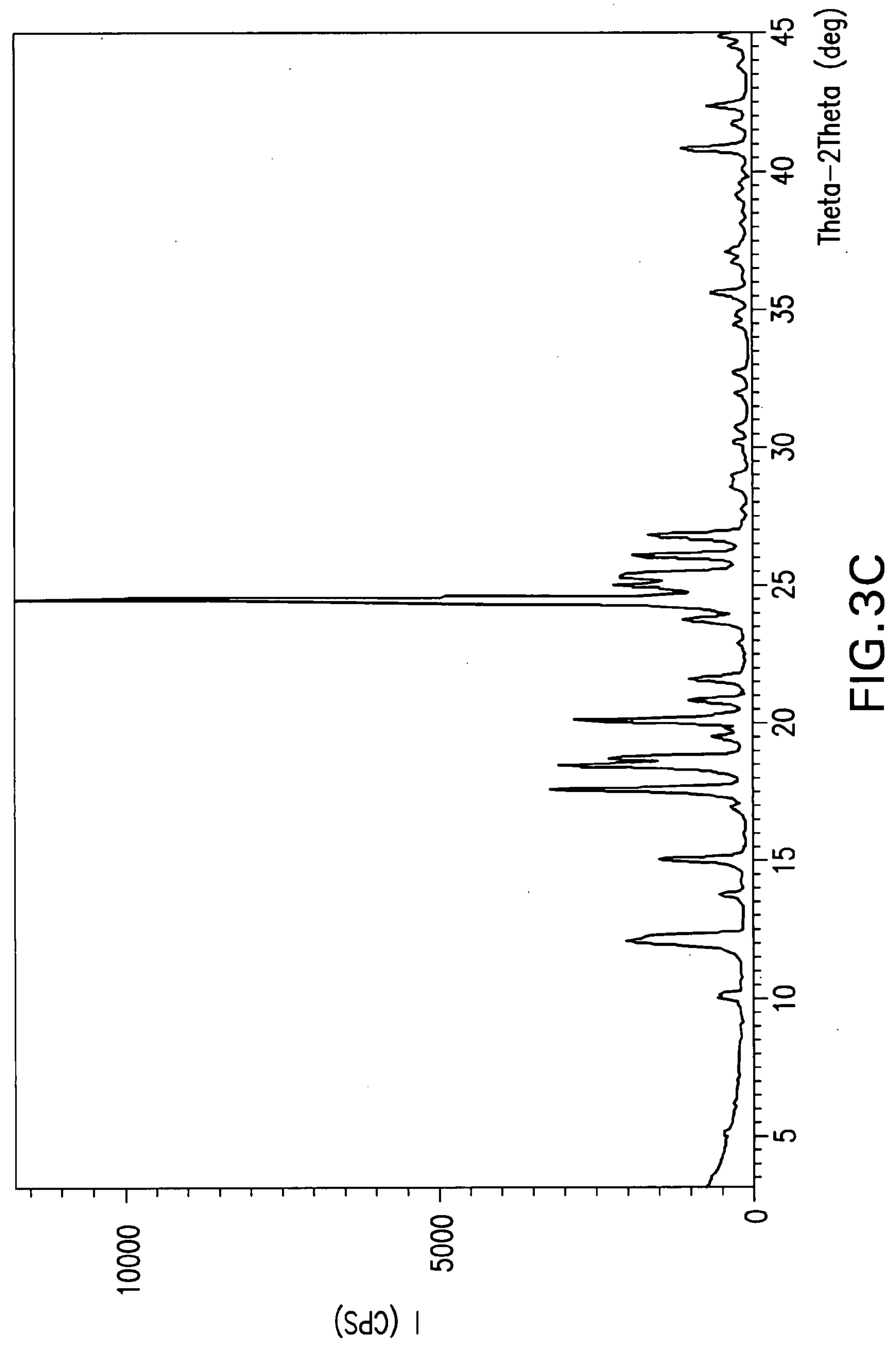


FIG.3C

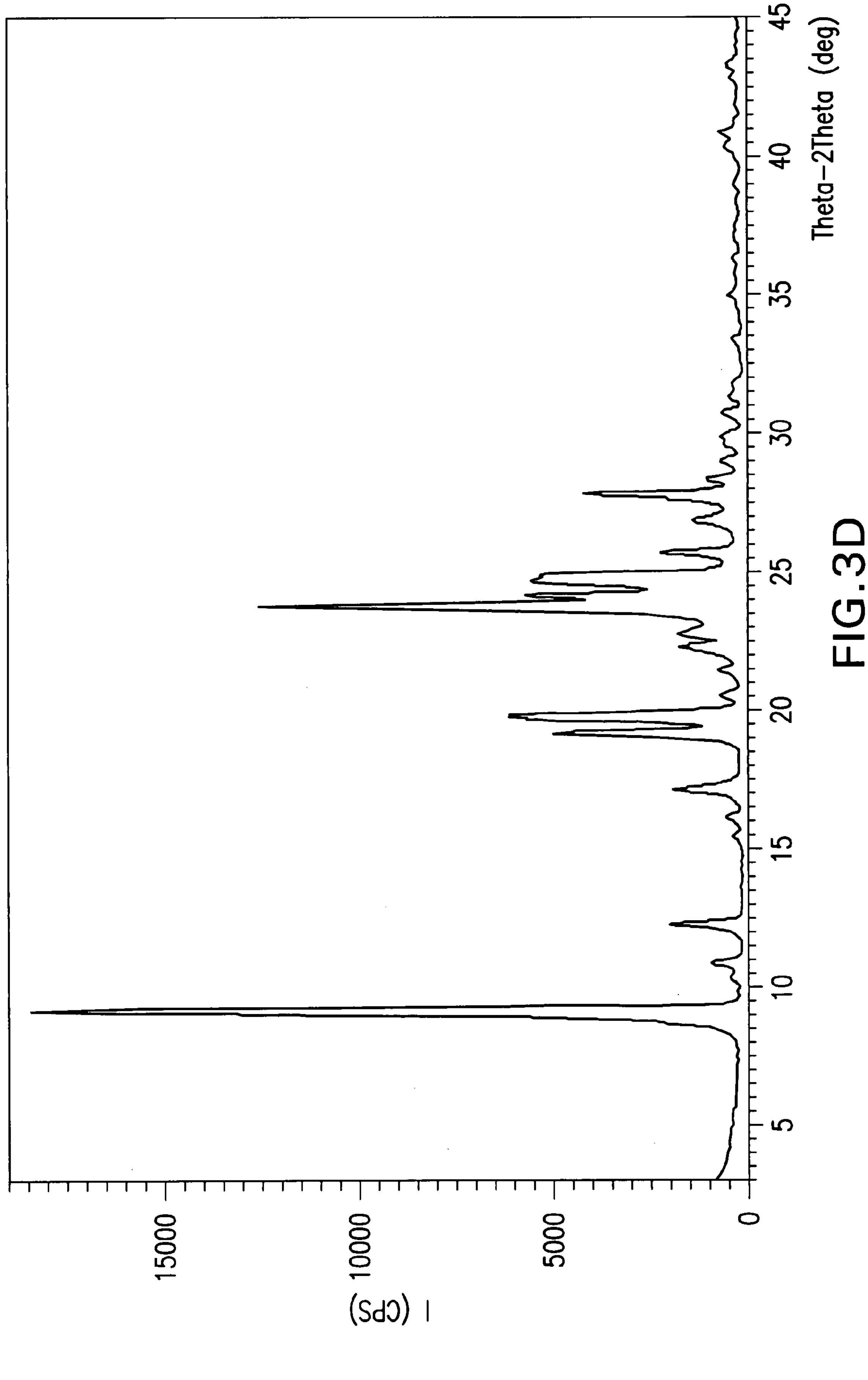
I (CPS)
0
5000
10000
15000
5
10
15
20
25
30
35
40
45
Theta-2Theta (deg)

FIG.3D

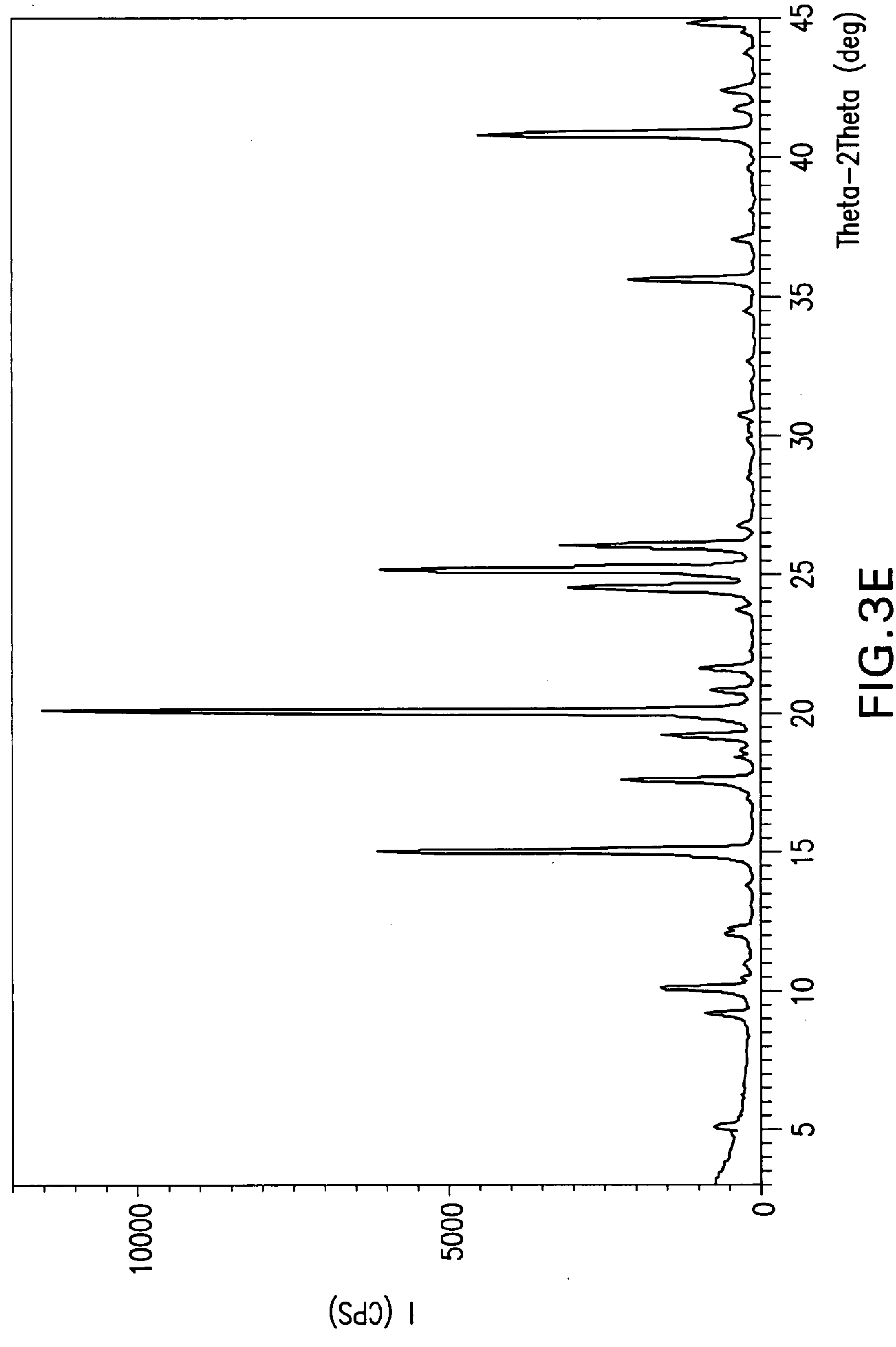


FIG.3E

Europäisches Patentamt
European Patent Office
Office européen des brevets

# EUROPEAN SEARCH REPORT

Application Number
EP 11 19 5407

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 2006/026260 A (MERCK & CO INC [US]; ATON PHARMA INC [US]; BELVEDERE SANDRO [US]; HAMB) 9 March 2006 (2006-03-09)<br>* See description, page 3 and claims 1, 10: hystone deacetylase inhibitors having a hydroxamic acid moiety, and their use for the treatment of cancer *<br>* See page 16, second paragraph: salts of the claimed compounds with zinc, copper, aluminum, iron *<br>----- | 1-9 | INV.<br>A61K31/16<br>A61K31/295<br>A61K33/26<br>A61K33/30<br>A61P35/00 |
| Y | WO 2005/110399 A (UNIV CALIFORNIA [US]; PUERTA DAVID T [US]; COHEN SETH M [US]; LEWIS JA) 24 November 2005 (2005-11-24)<br>* See page 2, line 15; page 8, line 23-24; page 19, line 7; claims 1, 3, 16, 22: compounds having histone deacetylase activity *<br>* See Zn chelates of histone deacetylase inhibitors in examples in pages 31, 32, 33 and in figures 1, 2, 3,4 , 5, 8, 14 *<br>* See page 12: pharmaceutical composition comprising zinc salts of the relevant compounds *<br>----- | 1-9 | TECHNICAL FIELDS SEARCHED (IPC)<br>A61K |
| Y | WO 01/18045 A (SLOAN KETTERING INST CANCER [US]; UNIV COLUMBIA [US]; PAVLETICH NIKOLA) 15 March 2001 (2001-03-15)<br>* See page 55, second paragraph: HDLP-Zn-SAHA co-crystals and preparation thereof *<br>----- | 1-9 | |
| E | US 2008/015352 A1 (PICCARIELLO THOMAS [US]) 17 January 2008 (2008-01-17)<br>* See claims 37 and 38, and in particular see "vorinostat" *<br>-----<br>-/-- | 1-9 | |

1

The present search report has been drawn up for all claims

| Place of search | Date of completion of the search | Examiner |
|---|---|---|
| Munich | 23 February 2012 | Veronese, Andrea |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 11 19 5407

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | MONNERET C: "Histone deacetylase inhibitors", EUROPEAN JOURNAL OF MEDICINAL CHEMISTRY, EDITIONS SCIENTIFIQUE ELSEVIER, PARIS, FR, vol. 40, no. 1, 1 January 2005 (2005-01-01), pages 1-13, XP004708959, ISSN: 0223-5234 * the whole document * ----- | 1-9 | |
| A | BEDALOV ANTONIO ET AL: "Identification of a small molecule inhibitor of Sir2p", PROCEEDINGS OF THE NATIONAL ACADEMY OF SCIENCES OF USA, NATIONAL ACADEMY OF SCIENCE, WASHINGTON, DC, US, vol. 98, no. 26, 18 December 2001 (2001-12-18), pages 15113-15118, XP002350537, ISSN: 0027-8424 * See abstract: Sir2p is a NAD+ dependent histone deacetylase * * See abstract: splitomicin as a Sir2p inhibitor * ----- | 1-9 | TECHNICAL FIELDS SEARCHED (IPC) |
| T | TRAPP JOHANNES ET AL: "THE ROLE OF NAD(+) DEPENDENT HISTONE DEACETYLASES (SIRTUINS) IN AGEING", CURRENT DRUG TARGETS, BENTHAM SCIENCE PUBLISHER, US, vol. 7, no. 11, 1 November 2006 (2006-11-01), pages 1553-1560, XP009082355, ISSN: 1389-4501 * See class III histone deacetylase, which are NAD-dependent, in contrast to classes I and II which are Zinc dependant * ----- -/-- | 1-9 | |

1

The present search report has been drawn up for all claims

| Place of search | Date of completion of the search | Examiner |
|---|---|---|
| Munich | 23 February 2012 | Veronese, Andrea |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 11 19 5407

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | GANTT STEPHANIE L ET AL: "Catalytic activity and inhibition of human histone deacetylase 8 is dependent on the identity of the active site metal ion.", BIOCHEMISTRY 16 MAY 2006, vol. 45, no. 19, 16 May 2006 (2006-05-16), pages 6170-6178, XP002550225, ISSN: 0006-2960<br>* See figures 4 and 5 and page 6175, second column last paragraph: complexes of HDAC8-Zn-SAHA and HDAC8-CU-SAHA *<br>----- | 1-9 | |

TECHNICAL FIELDS SEARCHED (IPC)

The present search report has been drawn up for all claims

1

| Place of search | Date of completion of the search | Examiner |
|---|---|---|
| Munich | 23 February 2012 | Veronese, Andrea |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 11 19 5407

This annex lists the patent family members relating to the patent documents cited in the above-mentioned European search report.
The members are as contained in the European Patent Office EDP file on
The European Patent Office is in no way liable for these particulars which are merely given for the purpose of information.

23-02-2012

| Patent document cited in search report | Publication date | Patent family member(s) | Publication date |
|---|---|---|---|
| WO 2006026260 A | 09-03-2006 | AU 2005280211 A1 | 09-03-2006 |
| | | CA 2576959 A1 | 09-03-2006 |
| | | CN 101035542 A | 12-09-2007 |
| | | EP 1784194 A1 | 16-05-2007 |
| | | JP 2008510821 A | 10-04-2008 |
| | | US 2009118291 A1 | 07-05-2009 |
| | | WO 2006026260 A1 | 09-03-2006 |
| WO 2005110399 A | 24-11-2005 | US 2007117848 A1 | 24-05-2007 |
| | | US 2008161362 A1 | 03-07-2008 |
| | | US 2009286987 A1 | 19-11-2009 |
| | | US 2010173952 A1 | 08-07-2010 |
| | | US 2012041032 A1 | 16-02-2012 |
| | | WO 2005110399 A2 | 24-11-2005 |
| WO 0118045 A | 15-03-2001 | AT 361316 T | 15-05-2007 |
| | | CA 2383885 A1 | 15-03-2001 |
| | | DE 60034688 T2 | 17-01-2008 |
| | | EP 1212357 A1 | 12-06-2002 |
| | | ES 2287033 T3 | 16-12-2007 |
| | | JP 2003518923 A | 17-06-2003 |
| | | WO 0118045 A1 | 15-03-2001 |
| US 2008015352 A1 | 17-01-2008 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4522811 A
- US 5608108 A
- US 5369108 A
- US 5932616 A
- US 5700811 A
- US 6087367 A
- US 6511990 B
- WO 0008048 A, H. Mori
- WO 9711366 A, P. Dulski
- WO 9848825 A


### Non-patent literature cited in the description


- **MARKS, P.A. et al.** *J. Natl. Cancer Inst.,* 2000, vol. 92, 1210-1215
- **BUTLER, L.M. et al.** *Cancer Res.,* 2000, vol. 60, 5165-5170
- **RICHON, V. M. et al.** *Proc. Natl. Acad. Sci., USA,* 1998, vol. 95, 3003-3007
- **YOSHIDA, M. et al.** *J. Biol. Chem.,* 1990, vol. 265, 17174-17179
- **YOSHIDA et al.** *J. Biol. Chem.,* 1990, vol. 265, 17174-17179
- **RICHON et al.** *Proc. Natl. Acad. Sci. USA,* 1998, vol. 95, 3003-3007
- **KOGHE et al.** *Biochem. Pharmacol.,* 1998, vol. 56, 1359-1364
- **ANDREWS et al.** *International J. Parasitology,* 2000, vol. 30, 761-768
- **QIU et al.** *Mol. Biol. Cell,* 2000, vol. 11, 2069-2083
- **KIM et al.** *Oncogene,* 1999, vol. 18, 2461-2470
- **SU et al.** *Cancer Research,* 2000, vol. 60, 3137-3142
- **KIJIMA et al.** *J Biol. Chem.,* 1993, vol. 268, 22429-22435
- **NAKAJIMA et al.** *Ex. Cell Res.,* 1998, vol. 241, 126-133
- **DARKIN-RATTRAY et al.** *Proc. Natl. Acad. Sci. USA,* 1996, vol. 93, 13143-13147
- **BOSCH et al.** *Plant Cell,* 1995, vol. 7, 1941-1950
- **COUSENS et al.** *J. Biol. Chem.,* 1979, vol. 254, 1716-1723
- **MCBAIN et al.** *Biochem. Pharm.,* 1997, vol. 53, 1357-1368
- **LEA ; TULSYAN.** *Anticancer Research,* 1995, vol. 15, 879-873
- **WANG et al.** *Cancer Research,* 1999, vol. 59, 2766-2799
- **GUAN et al.** *Cancer Research,* 2000, vol. 60, 749-755
- **SAITO et al.** *Proc. Natl. Acad. Sci. USA,* 1999, vol. 96, 4592-4597
- **FREY et al.** *Bioorganic & Med. Chem. Lett.,* 2002, vol. 12, 3443-3447
- **KWON et al.** *PNAS,* 1998, vol. 95, 3356-3361
- Cancer Chemotherapy: Principles and Practice. JB Lippincott, 1990
- **BLACK ; LIVINGSTON.** *Drugs,* 1990, vol. 39, 489-501
- *DRUGS,* vol. 39, 652-673
- **CHEN ; GREM.** *Curr. Opin. Oncol.,* 1992, vol. 4, 1089-1098
- **FRIEND, C. ; SCHER, W. ; HOLLAND, J. W. ; SATO, T.** *Proc. Natl. Acad. Sci. (USA,* 1971, vol. 68, 378-382
- **TANAKA, M. ; LEVY, J. ; TERADA, M. ; BRESLOW, R. ; RIFKIND, R. A. ; MARKS, P. A.** *Proc. Natl. Acad. Sci. (USA,* 1975, vol. 72, 1003-1006
- **REUBEN, R. C. ; WIFE, R. L. ; BRESLOW, R. ; RIFKIND, R. A. ; MARKS, P. A.** *Proc. Natl. Acad. Sci. (USA,* 1976, vol. 73, 862-866
- **ABE, E. ; MIYAURA, C. ; SAKAGAMI, H. ; TAKEDA, M. ; KONNO, K. ; YAMAZAKI, T. ; YOSHIKA, S. ; SUDA, T.** *Proc. Natl. Acad. Sci. (USA,* 1981, vol. 78, 4990-4994
- **SCHWARTZ, E. L. ; SNODDY, J. R. ; KREUTTER, D. ; RASMUSSEN, H. ; SARTORELLI, A. C.** *Proc. Am. Assoc. Cancer Res.,* 1983, vol. 24, 18
- **TANENAGA, K. ; HOZUMI, M. ; SAKAGAMI, Y.** *Cancer Res.,* 1980, vol. 40, 914-919
- **LOTEM, J. ; SACHS, L.** *Int. J. Cancer,* 1975, vol. 15, 731-740
- **SACHS, L.** *Nature (Lond.,* 1978, vol. 274, 535
- **METCALF, D.** *Science,* 1985, vol. 229, 16-22
- **SCHER, W. ; SCHER, B. M. ; WAXMAN, S.** *Exp. Hematol.,* 1983, vol. 11, 490-498

- **SCHER, W. ; SCHER, B. M. ; WAXMAN, S.** *Biochem. & Biophys. Res. Comm.,* 1982, vol. 109, 348-354
- **HUBERMAN, E. ; CALLAHAM, M. F.** *Proc. Natl. Acad. Sci. (USA,* 1979, vol. 76, 1293-1297
- **LOTTEM, J. ; SACHS, L.** *Proc. Natl. Acad. Sci. (USA,* 1979, vol. 76, 5158-5162
- **SCHWARTZ, E. L. ; SARTORELLI, A. C.** *Cancer Res.,* 1982, vol. 42, 2651-2655
- **TERADA, M. ; EPNER, E. ; NUDEL, U. ; SALMON, J. ; FIBACH, E. ; RIFKIND, R. A. ; MARKS, P. A.** *Proc. Natl. Acad. Sci. (USA,* 1978, vol. 75, 2795-2799
- **MORIN, M. J. ; SARTORELLI, A. C.** *Cancer Res.,* 1984, vol. 44, 2807-2812
- **SCHWARTZ, E. L. ; BROWN, B. J. ; NIERENBERG, M. ; MARSH, J. C. ; SARTORELLI, A. C.** *Cancer Res.,* 1983, vol. 43, 2725-2730
- **SUGANO, H. ; FURUSAWA, M. ; KAWAGUCHI, T. ; IKAWA, Y.** *Bibl. Hematol.,* 1973, vol. 39, 943-954
- **EBERT, P. S. ; WARS, I. ; BUELL, D. N.** *Cancer Res.,* 1976, vol. 36, 1809-1813
- **HAYASHI, M. ; OKABE, J. ; HOZUMI, M.** *Gann,* 1979, vol. 70, 235-238
- **MAI et al.** *OPPI Briefs,* 2001, vol. 33 (4), 391-394
- **STOWELL et al.** *J. Med. Chem.,* 1995, vol. 38 (8), 1411-1413
- **SPORN, M. B. ; ROBERTS, A. B. ; DRISCOLL, J. S.** Cancer: Principles and Practice of Oncology. J. B. Lippincott, 1985, 49
- **BREITMAN, T. R. ; SELONICK, S. E. ; COLLINS, S. J.** *Proc. Natl. Acad. Sci. USA,* 1980, vol. 77, 2936-2940
- **OLSSON, I. L. ; BREITMAN, T. R.** *Cancer Res.,* 1982, vol. 42, 3924-3927
- **MARKS, P. A. ; SHEFFERY, M. ; RIFKIND, R. A.** *Cancer Res.,* 1987, vol. 47, 659
- **ABE, E. ; MIYAURA, C. ; SAKAGAMI, H. ; TAKEDA, M. ; KONNO, K. ; YAMAZAKI, T. ; YOSHIKA, S. ; SUDA, T.** *Proc. Natl, Acad, Sci. (USA,* vol. 78, 4990-4994
- **RICHON, V.M. ; WEBB, Y. ; MERGER, R. et al.** *PNAS,* 1996, vol. 93, 5705-8
- **COHEN, L.A. ; AMIN, S. ; MARKS, P.A. ; RIFKIND, R.A. ; DESAI, D. ; RICHON, V.M.** *Anticancer Research,* 1999, vol. 19, 4999-5006
- **GRUNSTEIN, M.** *Nature,* 1997, vol. 389, 349-52
- **FINNIN, M.S. ; DONIGIAN, J.R. ; COHEN, A. et al.** *Nature,* 1999, vol. 401, 188-193
- **VAN LINT, C. ; EMILIANI, S. ; VERDIN, E.** *Gene Expression,* 1996, vol. 5, 245-53
- **ARCHER, S. SHUFEN ; M. SHEI, A. ; HODIN, R.** *PNAS,* 1998, vol. 95, 6791-96
- **DRESSEL, U. ; RENKAWITZ, R. ; BANIAHMAD, A.** *Anticancer Research,* 2000, vol. 20 (2A), 1017-22
- **PARKER ; VIGOROUX ; REED.** *AIChE J.,* 2000, 1290-99
- **NUNEZ ; ESPIELL.** *Chem.Eng.Sci.,* 1986, 2075-83
- **O. LEVENSPIEL.** Chemical Reaction Engineering. 373
- **M. VANNI.** *J. of Colloid and Interface Sci.,* 2000, 143-160
- **P. J. HILL ; K. M. NG.** *AIChE J.,* 1995, 1204-1216